# EUROPEAN PATENT SPECIFICATION

(11) **EP 0 876 333 B1**
(45) Date of publication and mention of the grant of the patent: **27.03.2002**
(21) Application number: 96939928.6
(22) Date of filing: 26.11.1996
(51) Int. Cl.: C07C 251/60, C07C 251/24, C07C 255/64, C07C 249/02, C07C 239/08

(54) **PROCESS FOR THE PREPARATION OF PESTICIDES**
VERFAHREN ZUR HERSTELLUNG VON PESTIZIDEN
PROCEDE DE PREPARATION DE PESTICIDES

(30) Priority: 07.12.1995 CH 346495
(43) Date of publication of application: 11.11.1998
(73) Proprietor: Bayer Aktiengesellschaft, 51368 Leverkusen (DE)
(72) Inventor: FAROOQ, Saleem, CH-4422 Arisdorf (CH); TRAH, Stephan, D-79111 Freiburg (DE); ZIEGLER, Hugo, CH-4108 Witterswil (CH); ZURFLÜH, René, 8180 Bülach (CH)
(86) International application number: PCT/EP96/05215
(87) International publication number: WO 97/20808

(56) References cited:
- EP-A- 0 617 014
- WO-A-95/18789
- WO-A-95/21153
- WO-A-95/21154
- WO-A-95/34526
- WO-A-96/11183
- WO-A-96/16026

## Description

The invention relates to a process for the preparation of compounds of the formula 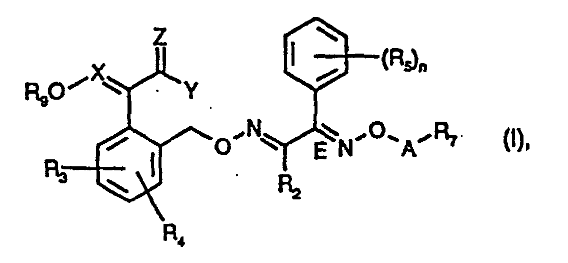 and, where appropriate, their tautomers, in each case in the free form or salt form, in which either
- X: is CH or N, Y is OR₁ and Z is O,
or
- X: is N, Y is NHR₈ and Z is O, S or S(=O);
- R₁: is C₁-C₄alkyl;
- R₂: is H, C₁-C₄alkyl, halogeno-C₁-C₄alkyl, C₃-C₆cycloalkyl or C₁-C₄alkoxymethyl;
- R₃ and R₄: independently of one another are H, C₁-C₄alkyl, C₁-C₄alkoxy, OH, CN, NO₂, a (C₁-C₄alkyl)₃-Si group, where the alkyl groups can be identical or different, halogen, (C₁-C₄alkyl)S(=O)ₘ, (halogeno-C₁-C₄alkyl)S(=O)ₘ, halogeno-C₁-C₄alkyl or halogeno-C₁-C₄alkoxy;
- R₅: is C₁-C₆alkyl, halogeno-C₁-C₆alkyl, C₁-C₆alkoxy, halogeno-C₁-C₆alkoxy; C₁-C₆-alkylthio, halogen-C₁-C₆alkylthio, C₁-C₆alkylsulfinyl, halogeno-C₁-C₆-alkylsulfinyl, C₁-C₆alkylsulfonyl, halogeno-C₁-C₆alkylsulfonyl, C₁-C₆alkoxy-C₁-C₆alkyl, halogeno-C₁-C₆alkoxy-C₁-C₆alkyl, C₁-C₆alkylthio-C₁-C₆alkyl, halogeno-C₁-C₆alkylthio-C₁-C₆alkyl, C₁-C₆alkylsulfinyl-C₁-C₆alkyl, halogeno-C₁-C₆-alkylsulfinyl-C₁-C₆alkyl, C₁-C₆-alkylsulfonyl-C₁-C₆alkyl, halogeno-C₁-C₆-alkylsulfonyl-C₁-C₆alkyl, C₁-C₆-alkylcarbonyl, halogeno-C₁-C₆-alkylcarbonyl, C₁-C₆-alkoxycarbonyl, halogeno-C₁-C₆-alkoxycarbonyl, C₁-C₆-alkylaminocarbonyl, C₁-C₄-alkoxyiminomethyl; di(C₁-C₆alkyl)-aminocarbonyl, where the alkyl groups can be identical or different; C₁-C₆-alkylaminothiocarbonyl; di(C₁-C₆alkyl)aminothiocarbonyl, where the alkyl groups can be identical or different; C₁-C₆-alkylamino, di(C₁-C₆alkyl)-amino, where the alkyl groups can be identical or different; halogen, NO₂, CN, SF₅, thioamido, thiocyanatomethyl; an unsubstituted or mono- to tetrasubstituted C₁-C₄alkylenedioxy group, where the substituents are selected from the group consisting of C₁-C₄alkyl and halogen; or QR₆, where, If n is greater than 1, the radicals R₅ can be identical or different;
- R₆: is C₂-C₆alkenyl or C₂-C₆ alkynyl which are unsubstituted or substituted by 1 to 3 halogen atoms; (C₁-C₄alkyl)₃Si, where the alkyl groups can be identical or different; CN; or an unsubstituted or mono- to pentasubstituted C₃-C₆cycloalkyl, aryl or heterocyclyl group, where the substituents are selected from the group consisting of halogen, C₁-C₆alkyl, halogeno-C₁-C₆alkyl, C₁-C₆alkoxy, halogeno-C₁-C₆alkoxy, phenoxy, naphthoxy and CN;
- A: either is a direct bond, C₁-C₁₀alkylene, -C(=O)-,-C(=S)- or halogeno-C₁-C₁₀alkylene and R₇ is a radical R₁₀,
or is C₁-C₁₀alkylene, -C(=O)-,-C(=S)- or halogeno-C₁-C₁₀alkylene and
R₇ is OR₁₀, N(R₁₀)₂, where the radicals R₁₀ can be identical or different, or -S(=O)_{q}R₁₀;
- R₈: is H or C₁-C₄alkyl;
- R₉: is methyl, fluoromethyl or difluoromethyl;
- R₁₀: is H; an unsubstituted or substituted C₁-C₆alkyl, C₂-C₆alkenyl or C₂-C₆alkynyl group, where the substituents are selected from the group consisting of halogen; (C₁-C₄alkyl)₃Si, where the alkyl groups can be identical or different; C₃-C₆cyclo-alkyl, which is unsubstituted or substituted by halogen; C₁-C₆alkoxycarbonyl, which is unsubstituted or substituted by halogen; unsubstituted or substituted aryl, where the substituents are selected from the group consisting of halogen, halogeno-C₁-C₄alkyl and CN; a (C₁-C₄alkyl)₃Si group, where the alkyl groups can be identical or different; C₃-C₆cycloalkyl, which is unsubstituted or substituted by halogen; C₁-C₆alkoxycarbonyl which is unsubstituted or substituted by halogen; or an unsubstituted or substituted aryl or heterocyclyl group, where the substituents are selected from the group consisting of halogen and halogeno-C₁-C₄alkyl;
- Q: is a direct bond, C₁-C₈alkylene, C₂-C₆alkenylene, C₂-C₆alkynylene, O, O(C₁-C₆alkylene), (C₁-C₆alkylene)O, S(=O)ₚ, S(=O)ₚ(C₁-C₆alkylene) or (C₁-C₆alkylene)S(=O)ₚ;
- m: is 0, 1 or 2;
- n: is 0, 1, 2, 3, 4 or 5;
- p: is 0, 1 or 2; and
- q: is 0, 1 or 2,
and the C=N double bond marked with E has the E configuration, which comprises
c) reacting a compound of the formula 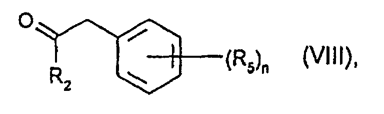 in which R₂, R₅ and n are as defined for formula (I), or a possible tautomer thereof, in each case in the free form or in salt form with a C₁-C₆alkyl nitrite in order to obtain a compound of the formula 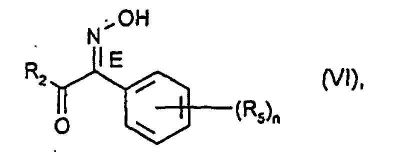 in which R₂, R₅ and n are as defined for formula (I) and the C=N double bond marked with E has the E-configuration, or a possible tautomer thereof, in each case in the free form or in salt form;
b1) converting the said compound of the formula (VI) into a compound of the formula 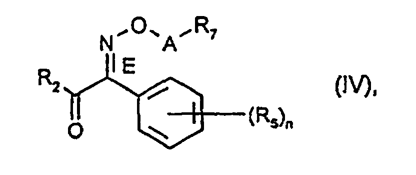 in which A, R₂, R₅, R₇ and n are as defined for formula (I) and the C=N double bond marked with E has the E-configuration, or a possible tautomer thereof, in each case in the free form or in the salt form, by reacting the compound of the formula (VI) with a compound of the formula

R₇-A-X₂ (VII),

in which A and R₇ are as defined for formula (I) and X₂ is a leaving group, and, in order to obtain a compound of the formula (I), *either*
a2) reacting a compound of the formula (IV) with a compound of the formula 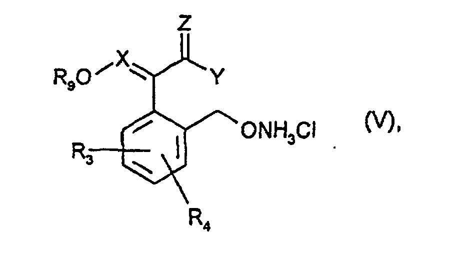 in which X, Y, Z, R₃, R₄ and R₉ are as defined for formula (I), or, if appropriate, a tautomer thereof, in each case in the free form or in salt form; *or*
b2) converting the compound of the formula (IV) into a compound of the formula 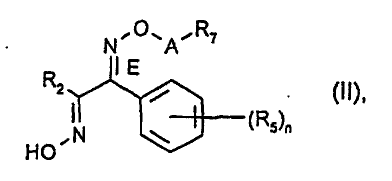 in which A, R₂, R₅, R₇ and n are as defined for formula (I) and the C=N double bond marked with E has the E-configuration, or a possible tautomer thereof, in each case in the free form or in salt form, by reacting the compound of the formula (IV) with hydroxylamine or a salt thereof; and
a1) reacting a compound of the formula (II) with a compound of the formula 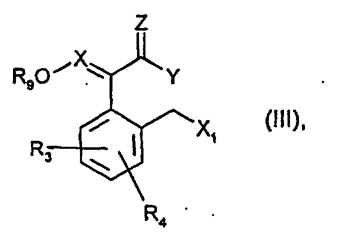 in which X, Y, Z, R₃, R₄ and R₉ are as defined for formula (I) and X₁ is a leaving group, or a tautomer thereof, in each case in the free from or in salt form,
the E isomers of the compounds of the formulae II, IV and VI, or a tautomer thereof, in each case in the free form or in salt form, a process for their preparation and their use for the preparation of compounds of the formula I.

In WO 95/21154 phenyl acelic acide derivatives, process and intermediate products for their production and agents containing them are described.

The compounds of the formula I are known pesticides. The processes known to date for their preparation give mixtures of E and Z isomers in respect of the C=N double bond marked with E in formula I of different composition, depending on the process. Since the biological properties of the E isomers are in each case found to be superior to those of the mixtures and of the Z isomers, there is a need to develop preparation processes for compounds of the formula I having the isomerically pure E configuration. This object is achieved by the preparation process according to the invention.

Unless defined differently, the general terms used above and below are defined as follows.

Carbon-containing groups and compounds in each case contain 1 up to and including 8, preferably 1 up to and including 6, in particular 1 up to and including 4, especially 1 or 2, carbon atoms.

Alkyl - as a group per se and as a structural element of other groups and compounds, such as of halogenoalkyl, alkoxy, alkylthio, alkylsulfinyl, alkylsulfonyl, alkylcarbonyl, alkoxycarbonyl, halogenoalkoxycarbonyl, alkylaminocarbonyl,alkoxyiminomethyl, alkylaminothiocarbonyl and alkylamino - is, in each case taking into due consideration the number, included from case to case, of carbon atoms contained in the corresponding group or compound, either straight-chain, i.e. methyl, ethyl, propyl, butyl, pentyl or hexyl, or branched, for example isopropyl, isobutyl, sec-butyl, tert-butyl, isopentyl, neopentyl or isohexyl.

Alkenyl - as a group per se and as a structural element of other groups and compounds, such as of halogenoalkenyl - is, in each case under due consideration of the number, included from case to case, of carbon atoms contained in the corresponding group or compound, either straight-chain, for example vinyl, 1 -methylvinyl, allyl, 1-butenyl or 2-hexenyl, or branched, for example iso-propenyl.

Alkynyl - as a group per se and as a structural element of other groups and compounds, such as of halogenoalkynyl - is, in each case under due consideration of the number, included from case to case, of carbon atoms contained in the corresponding group or compound, either straight-chain, for example propargyl, 2-butynyl or 5-hexynyl, or branched, for example 2-ethynylpropyl or 2-propargylisopropyl.

C₃-C₆cycloalkyl is cyclopropyl, cyclobutyl, cyclopentyl or cyclohexyl.

Alkylene - as a group per se and as a structural element of other groups and compounds, such as of O(alkylene), (alkylene)O, S(=O)ₚ(alkylene), (alkylene)S(=O)ₚ or alkylenedioxy - is, in each case under due consideration of the number, included from case to case, of carbon atoms contained in the corresponding group or compound, either straight-chain, for example -CH₂CH₂-, -CH₂CH₂CH₂- or -CH₂CH₂CH₂CH₂-, or branched, for example -CH(CH₃)-, -CH(C₂H₅)-, -C(CH₃)₂-, - CH(CH₃)CH₂- or -CH(CH₃)CH(CH₃)-.

Alkenylene is, in each case under due consideration of the number, from case to case, of carbon atoms contained in the corresponding compound, either straight-chain, for example vin-1,2-ylene, all-1,3-ylene, but-1-en-1,4-ylene or hex-2-en-1,6-ylene, or branched, for example 1-methylvin-1,2-ylene.

Alkynylene is, in each case under due consideration of the number, from case to case, of carbon atoms contained in the corresponding compound, either straight-chain, for example propargylene, 2-butynylene or 5-hexynylene, or branched, for example 2-ethynylpropylene or 2-propargylisopropylene.

Aryl is phenyl or naphthyl, in particular phenyl.

Heterocyclyl is a 5- to 7-membered aromatic or non-aromatic ring having one to three heteroatoms, which are selected from the group consisting of N, O and S. 5- and 6-membered rings which contain a nitrogen atom as a heteroatom and, if appropriate, a further heteroatom, preferably nitrogen or sulfur, in particular nitrogen, are preferred.

Halogen - as a group per se and as a structural element of other groups and compounds, such as of halogenoalkyl, halogenoalkenyl and halogenoalkynyl - is fluorine, chlorine, bromine or iodine, especially fluorine, chlorine or bromine, in particular fluorine or chlorine, very especially fluorine.

Halogen-substituted carbon-containing groups and compounds, such as halogenoalkyl, halogenoalkenyl or halogenoalkynyl, can be partly halogenated or perhalogenated, and in the case of polyhalogenation, the halogen substituents can be identical or different. Examples of halogenoalkyl- as a group per se and as a structural element of other groups and compounds, such as of halogenoalkenyl- are methyl which is mono- to trisubstituted by fluorine, chlorine and/or bromine, such as CHF₂ or CF₃; ethyl which is mono- to pentasubstituted by fluorine, chlorine and/or bromine. such as CH₂CF₃, CF₂CF₃, CF₂CCl₃, CF₂CHCl₂, CF₂CHF₂, CF₂CFCl₂, CF₂CHBr₂, CF₂CHClF, CF₂CHBrF or CClFCHClF; propyl or isopropyl which is mono- to heptasubstituted by fluorine, chlorine and/or bromine, such as CH₂CHBrCH₂Br, CF2CHFCF₃, CH₂CF₂CF₃ or CH(CF₃)₂; and butyl or one of its isomers which is mono- to nonasubstituted by fluorine, chlorine and/or bromine, such as CF(CF₃)-CHFCF₃or CH₂(CF₂)₂CF₃. Halogenoalkenyl is, for example, CH₂CH=CHCl, CH₂CH=CCl₂, CH₂CF=CF₂ or CH₂CH=CHCH₂Br. Halogenoalkynyl is, for example, CH₂C≡CF, CH₂C≡CCH₂Cl or CF₂CF₂C≡CCH₂F.

Some compounds I to VI and VIII can be present as tautomers, as is familiar to the expert, in particular if AR₇ is H. Compounds I above and below are therefore also to be understood as meaning corresponding tautomers, even if the latter are not mentioned specifically in each case.

Compounds I to VI and VIII which contain at least one basic centre, can form, for example, acid addition salts. These are formed, for example, with strong inorganic acids, such as mineral acids, for example perchloric acid, sulfuric acid, nitric acid, nitrous acid, a phosphoric acid, or a hydrogen halide acid, with strong inorganic carboxylic acids, such as C₁-C₄alkanecarboxylic acids which are unsubstituted or substituted, for example by halogen, for example acetic acid, such as dicarboxylic acids which are saturated or unsaturated, for example oxalic, malonic, succinic, maleic, fumaric or phthalic acid, such as hydroxycarboxylic acids, for example ascorbic, lactic, malic, tartaric or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds I with at least one acid group can furthermore form salts with bases. Suitable salts with bases are, for example, metal salts such as alkali metal or alkaline earth metal salts, for example sodium, potassium or magnesium salts, or salts with ammonia or an organic amine, such as morpholine, piperidine, pyrrolidine, a mono-, di- or tri-lower alkylamine, for example ethyl-, diethyl-, triethyl- or dimethyl-propyl-amine, or a mono-, di- or trihydroxy-lower alkylamine, for example mono-, di- or triethanolamine. Furthermore, where appropriate, corresponding inner salts can be formed. Agrochemically advantageous salts are preferred in the context of the invention; however, salts which have disadvantages for agrochemical uses, for example salts which are toxic to bees or fish, which are employed, for example, for isolation or purification of free compounds I or agrochemically usable salts thereof, are also included. Compounds of the formulae I to VI and VIII in the free form and in the form of their salts are also to be understood above and below as meaning the corresponding salts or the free compounds I to VI and VIII. The same applies to tautomers of compounds of the formulae I to VI and VIII and salts thereof. In general, the free form is in each case preferred.

The reactions described above and below are carried out in a manner known per se, for example in the absence or usually in the presence of a suitable solvent or diluent or a mixture thereof, the reaction being carried out, as required, with cooling, at room temperature or with heating, for example in a temperature range from about -80°C up to the boiling point of the reaction medium, preferably from about 0°C up to about 150°C, and, if necessary, in a closed vessel, under pressure, in an inert gas atmosphere and/or under anhydrous conditions. Particularly advantageous reaction conditions can be seen from the examples.

The starting materials mentioned above and below, which are used for the preparation of the compounds I, in each case in the free form or in salt form, are known or can be prepared by methods known per se, for example in accordance with the following statements.

### Variants a1/a2)

Suitable leaving groups X₁ in compounds III are, for example, hydroxyl, C₁-C₈alkoxyl, halogeno-C₁-C₈alkoxy, C₁-C₈alkanoyloxy, mercapto, C₁-C₈alkylthio, halogeno-C₁-C₈alkylthio, C₁-C₈alkanesulfonyloxy, halogeno-C₁-C₈alkanesulfonyloxy, benzenesulfonyloxy, toluenesulfonyloxy and halogen, preferably toluenesulfonyloxy, trifluoromethanesulfonyloxy and halogen, in particular halogen.

Suitable bases for facilitating the reaction are, for example, alkali metal or alkaline earth metal hydroxides, hydrides, amides, alkanolates, acetates, carbonates, dialkylamides or alkylsilylamides, alkylamines, alkylenediamines, N-alkylated or non-alkylated, saturated or unsaturated cycloalkylamines, basic heterocyclic compounds, ammonium hydroxides and carbocyclic amines. Examples are sodium hydroxide, hydride, amide, methanolate, acetate and carbonate, potassium tert-butanolate, hydroxide, carbonate, and hydride, lithium diisopropylamide, potassium bis(trimethylsilyl)amide, calcium hydride, triethylamine, diisopropyl-ethyl-amine, triethylenediamine, cyclohexylamine, N-cyclohexyl-N,N-dimethyl-amine, N,N-diethylaniline, pyridine, 4-(N,N-dimethylamino)pyridine, quinuclidine, N-methylmorpholine, benzyl-trimethyl-ammonium hydroxide and 1,5-diazabicyclo[5.4.0]undec-5-ene (DBU).

The reaction partners can be reacted with one another as such, i.e. without addition of a solvent or diluent, for example in the melt. However, the addition of an inert solvent or diluent or of a mixture thereof is usually advantageous. Examples of such solvents or diluents are: aromatic, aliphatic and alicyclic hydrocarbons and halogenohydrocarbons, such as benzene, toluene, xylene, mesitylene, tetralin, chlorobenzene, dichlorobenzene, bromobenzene, petroleum ether, hexane, cyclohexane, methylene chloride, chloroform, carbon tetrachloride, dichloroethane, trichloroethene or tetrachloroethene; esters, such as ethyl acetate; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethyl ether, dimethoxydiethyl ether, tetrahydrofuran or dioxane; ketones, such as acetone, methyl ethyl ketone or methyl isobutyl ketone; alcohols, such as methanol, ethanol, propanol, isopropanol, butanol, ethylene glycol or glycerol; amides, such as N,N-dimethylformamide, N,N-diethylformamide, N,N-dimethylacetamide, N-methylpyrrolidone or hexamethylphosphoric acid triamide; nitrites such as acetonitrile or propionitrile; and sulfoxides, such as dimethyl sulfoxide. If the reaction is carried out in the presence of a base, bases employed in excess, such as triethylamine, pyridine, N-methylmorpholine or N,N-diethylaniline, can also serve as the solvent or diluent.

The reaction is advantageously carried out in a temperature range from about 0°C up to about 180°C, preferably from about 10°C up to about 80°C, in many cases in the range between room temperature and the reflux temperature of the reaction mixture.

The reaction is preferably carried out under normal pressure.

The reaction can be carried out without an inert gas atmosphere; preferably, however, it is carried out under an inert gas atmosphere, for example nitrogen or argon, in particular nitrogen.

The reaction time is not critical; a reaction time of about 0.1 to about 24 hours, in particular about 0.5 to about 2 hours, is preferred.

The product is isolated by customary methods, for example by filtration, crystallization, distillation or chromatography or any suitable combination of these processes.

In a preferred embodiment of variants a1/a2), a compound II is reacted with a compound III at 0°C to 80°C, preferably 10° C to 30° C, in an inert solvent, preferably an amide, in particular N,N-dimethylformamide in the presence of a metal hydride, preferably sodium hydride.

Particularly preferred conditions for the reaction are described in Examples H1d) and H 3f).

The compounds of the formula III are known or can be prepared analogously to known compounds.

The compounds I are known. However, their preparation according to the prior art has a large number of serious industrial, ecological, economic and other disadvantages.

Thus, in the preparation processes according to the prior art, as a rule E/Z isomer mixtures with respect to the C=N double bond marked with E in formula I are obtained. Since the biological properties of the E isomers are in each case found to be superior to those of the mixtures and of the Z isomers in each case, the processes according to the prior art have the significant disadvantage that products are produced which are either significantly less active as E/Z mixtures or from which the Z isomers must be removed in order to increase their biological activity, which means that many unnecessary handling operations must be carried out for separation of isomers, which has the effect of being very time-consuming, blocks valuable production lines for a long time and is associated with high additional energy costs. The removal of the less active Z isomer also leads to additional enormous losses in yield, which in turn not only is problematic and ecologically disadvantageous, but also renders the process according to the prior art much more expensive and consequently economically of no interest. The industrial, ecological, economic and other disadvantages of the processes according to the prior art are not limited to those described above, these latter being intended to serve only as a few examples of the large number of disadvantages of the processes according to the prior art. The disadvantages of the processes according to the prior art cause serious problems even when the processes are carried out on a laboratory scale. When the processes are carried out on a larger scale, these disadvantages intensify considerably. In the end, however, the aim is to carry out a specific process on an industrial scale if this process is to be suitable for preparing products for agrochemical purposes.

According to the process of the present invention, the compounds I are prepared by reaction of the compound II with a compound III or by reaction of the compound IV with a compound V. These processes according to the invention have extremely surprising industrial, ecological, economic and other advantages compared with the processes from the prior art. Since the compounds II or, respectively, IV are present in the preparation process according to the invention as pure E isomers in respect of the C=N double bond marked with E, only the E isomer of the compounds I is produced in the present process, which has the effect of an enormous saving in time and at the same time a high saving in cost and energy, since no valuable production lines are blocked for a long time for separation of the isomers, and at the same time the amount of biologically more active E isomer produced by per unit time is much higher than in the processes according to the prior art. The resources such as starting products and energy are consequently utilized to the optimum in the present process, which not only very greatly simplifies the process and renders it ecologically advantageous, but consequently renders it cheaper and therefore of greater economic interest. This means that all the disadvantages of the processes according to the prior art which can be attributed to the formation of E/Z isomers are avoided. The industrial, ecological, economic and other advantages of the process according to the invention are not limited only to those described above, these latter being intended to serve only as a few examples of the large number of advantages inherent in this process. Due to all the abovementioned advantages of the present process, the serious problems which occur in the processes according to the prior art are avoided even at the stage of a laboratory process. If the present process is used on a larger scale, these advantages prove to be even much more significant, which has the effect that these advantages first allow the process to be used on an industrial scale.

For this reason, all the industrial, ecological, economic and other disadvantages of the processes according to the prior art are surprisingly advantageously overcome in the preparation of compounds 1 by the present process.

### Variant b)

The process according to variant b) is carried out by first reacting compound VI with compound VII, if appropriate further reacting the resulting product IV, if appropriate after isolation, with hydroxylamine or a salt thereof, and further reacting the resulting products II or, respectively, IV, if appropriate after isolation, in accordance with variants a1/a2), for example in the manner described above, to give the compounds I.

Suitable leaving groups X₂ in the compounds VII are, for example, those which are mentioned as examples for X₁ in variants a1/a2).

Suitable bases for facilitating the reaction are, for example, those which are mentioned in variants a1/a2).

The reaction partners can be reacted with one another as such, i.e. without addition of a solvent or diluent, for example in the melt. However, the addition of an inert solvent or diluent or of a mixture thereof is usually advantageous. Examples of such solvents or diluents are those mentioned in variants a1/a2).

The reaction is advantageously carried out in a temperature range from about 0°C to about 180°C, preferably from about 10°C to about 80°C, in many cases in the range between room temperature and the reflux temperature of the reaction mixture.

The reaction is preferably carried out under normal pressure.

The reaction can be carried out without an inert gas atmosphere; preferably, however, it is carried out under an inert gas atmosphere, for example nitrogen or argon, in particular nitrogen.

The reaction time is not critical; a reaction time of about 0.1 to about 24 hours, in particular about 0.5 to about 5 hours, is preferred.

The product is isolated by customary methods, for example filtration, crystallization, distillation or chromatography or any suitable combination of these processes.

In a preferred embodiment of variant b), a compound VI is reacted with a compound VII at 0°C to 80°C, preferably 10°C to 60°C, in an inert solvent, preferably a nitrile, in particular acetonitrile, in the presence of a metal carbonate, preferably potassium carbonate, and the compound IV thus obtainable is then further reacted, preferably in accordance with method a2).

Particularly preferred conditions for the reaction are described in Examples H 1b) to 1d) and H 3d) to 3f).

The compounds of the formula VII are known or can be prepared analogously to known compounds.

The present process according to the invention of variant b), which in principle is an advantageous combination of an O-alkylation reaction with process variants a1/a2) according to the invention, has all the great advantages compared with the prior art which have already been discussed above for the process according to the invention of variants a1/a2). In particular, the process of variant b) ensures that the E configuration of the C=N double bond marked with E in compound VI is retained. Furthermore, however, the process according to the invention of variant b) also has further industrial, ecological, economic and other advantages which are connected with the specific property that the intermediate product IV initially formed is not purified but is directly further processed as the moist crude product, in the case of intermediate isolation, or in situ in the reaction mixture, if it is not isolated. This missing purification step on the intermediate product mentioned is of advantage, for example, in as much as it is not necessary to dry it, which not only saves energy and further resources, but also enormously increases the safety of the preparation process, since the possible danger of a dust explosion of the dry intermediate product is averted completely. The savings in resources are even greater if the intermediate product is further reacted without purification, since, for example, no additional solvents are consumed for the recrystallization. The process of variant b) is of particular advantage compared with the individual process steps of the alkylation reaction of variants a1/a2) carried out in that the total reaction time in the process of variant b) is much shorter, which consequently leads to a much higher production of reaction product I per unit time and therefore to a much more efficient utilization of the valuable production lines. Furthermore, the total yield of reaction product I is surprisingly good when the process of variant b) is employed, and, compared with the combined yields of the individual process steps of the alkylation reaction and variants a1/a2) carried out, is in the same percentage range or even better. The industrial, ecological, economic and other advantages of the process according to the invention of variant b) are not limited to those described above, these latter being intended to serve only as a few examples of the large number of advantages inherent in the process according to the invention of variant b).

By using process variant b) according to the invention for preparation of the compounds I, a large number of industrial, ecological, economic and other advantages can therefore surprisingly be utilized efficiently.

### Variant c)

The process according to variant c) is carried out by first reacting compound VIII with an alkyl nitrite and further reacting the resulting product VI, if appropriate after isolation, in accordance with variant b), for example in the manner described above, to give the compounds I.

Suitable bases for facilitating the reaction are, for example, those which are mentioned in variants a1/a2).

The reaction partners can be reacted with one another as such, i.e. without addition of a solvent or diluent, for example in the melt. However, the addition of an inert solvent or diluent or of a mixture thereof is usually advantageous. Examples of such solvents or diluents are those mentioned in variants a1/a2).

The reaction is advantageously carried out in a temperature range from about 0°C to about 180°C, preferably from about 0°C to about 60°C, in many cases in the range between room temperature and the reflux temperature of the reaction mixture.

The reaction is preferably carried out under normal pressure.

The reaction can be carried out without an inert gas atmosphere; preferably, however, it is carried out under an inert gas atmosphere, for example nitrogen or argon, in particular nitrogen.

The reaction time is not critical; a reaction time of about 0.1 to about 24 hours, in particular about 0.5 to about 3 hours, is preferred.

The product is isolated by customary methods, for example filtration, crystallization, distillation or chromatography or any suitable combination of these processes.

In a preferred embodiment of variant c), a compound VIII is reacted with an alkyl nitrite at 0°C to 80°C, preferably 0°C to 40°C, in an inert solvent, preferably an alcohol, in particular methanol, in the presence of a metal alcoholate, preferably sodium methanolate, and the compound VI thus obtainable is then further reacted, preferably in accordance with method b).

Particularly preferred conditions for the reactions are described in Examples H 3d) to 3f).

The compounds of the formula VIII are known or can be prepared analogously to known compounds.

The present process according to the invention of variant c), which in principle is an advantageous combination of an oximation reaction with process variants a1/a2) and b) according to the Invention, has all the great advantages compared with the prior art which have already been discussed above for the processes according to the invention of variants a1/a2) and b). Furthermore, the present oximation process for the preparation of the compounds VI surprisingly results exclusively in the E configuration of the C=N double bond marked with E In formula VI. It Is thus ensured that the particular starting products II, IV or, respectively, VI In the subsequent processes according to the Invention for the preparation of the compounds I, for example in process variants a1/a2) and b), are pure E isomers.

A large number of industrial, ecological, economic and other advantages can therefore surprisingly be utilized efficiently by using process variants c) according to the Invention for the preparation of the compounds of the formula I.

The present Invention furthermore relates to a process for the preparation of a compound of the formul 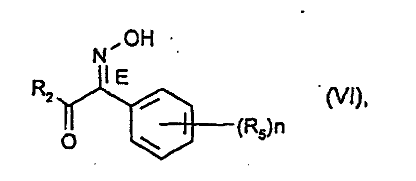 in which R₂, R₅ and n are as defined for formula (I) and the C=N double bond marked with E has the E-configuration, which comprises reacting the compound of the formula (VIII), as defined above, with a C₁-C₆alkyl nitrite (process c)),
a process for the preparation of a compound of the formula 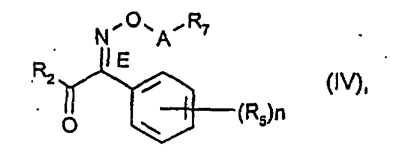 in which A, R₂, R₅, R₇ and n are as defined for formula (I) and the C=N double bond marked with E hastheE-configuration, which comprises.
c) reacting a compound of the formula (VIII) as defined above with a C₁-C₆alkyl nitrite in order to obtain a compound of the formula (VI) as defined above; and
b1) converting the said compound of the formula (VI) into a compound of the formula (IV), as defined above by reacting the compound of the formula (VI) with a compound of the formula (VII), as defined above, and
a process for the preparation of a compound of the formula 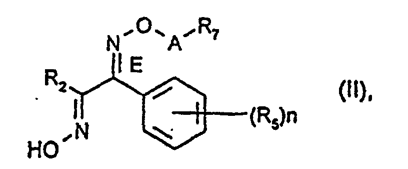 in which A, R₂, R₅, R₇ and n are as defined for formula (I) and the C=N double bond marked with E has the E-configuration, which comprises
c) reacting a compound of the formula (VIII) as defined above, with a C₁-C₆alkyl nitrite in order to obtain a compound of the formula (VI), as defined above;
b1) converting the said compound of the formula (VI) into a compound of the formula (IV), as defined above, by reacting the compound of the formula (VI) with a compound of the formula (VII), as defined above; and
b2) converting the compound of the formula (IV) into a compound of the formula (II) by reacting the compound of the formula (IV) with hydroxylamine or a salt thereof.

The process conditions for the preparation of these intermediate products can be seen from the abovementioned processes a), b) and c).

### Preparation Examples

### Example H1: Methyl 2-[[[(1-methyl-2-phenyl-2-E-[(2-propynyl)oxyimino]ethylidene)amino]oxy]methyl]α-(methoxymethylene)-phenylacetate (Compound 1.16)

### H1a) 1-Phenyl-1,2-propanedione 1-E-oxime

69.7 g of a 30 % solution of sodium methylate in methanol are added dropwise to a solution of 40.2 g of 1-phenyl-2-propanone and 36.1 g of isopentyl nitrite in 460 ml of methanol at 20-25°, while cooling. The reaction mixture is then further stirred at room temperature for 1 hour. After the solution has been concentrated in vacuo, the residue is dissolved in 600 ml of water, the solution is acidified with 10 % hydrochloric acid, the product which precipitates out is filtered off and dissolved in ethyl acetate and the organic phase is washed twice with water, dried with sodium sulfate and evaporated in vacuo. The residue is stirred up in hexane and filtered. The title product is thus obtained with a melting of 168-70°C.

### H1b) 1-Phenyl-1,2-propanedione 1-E-[(2-propynyl)oxime]

A mixture of 14 g of 1-phenyl-1,2-propanedione 1-E-oxime, 11.9 g of 1-bromo-2-propyne, 13.8 g of potassium carbonate and 0.5 g of potassium iodide in 170 ml of acetonitrile is stirred at 50° for 2 hours, the solvent is then distilled off in vacuo and the residue is dissolved again in ethyl acetate. The organic phase is washed in each case twice with water and saturated sodium chloride solution, dried with sodium sulfate and evaporated in vacuo. After recrystallization of the residue from hexane, 1-phenyl-1,2-propanedione 1-E-[(2-propynyl)oxime] is obtained with a melting point of 54-56°C.

### H1d) 1-Phenyl-1,2-propanedione 1-E-[(2-propynyl)oxime]-2-oxime

A mixture of 14.3 g of 1-phenyl-1,2-propanedione 1-E-[(2-propynyl)oxime], 10.3 g of hydroxylamine hydrochloride and 11.7 g of pyridine in 230 ml of ethanol is boiled under reflux for 1 hour and then concentrated in vacuo, and 800 ml of water are added to the residue. The product which has precipitated out is filtered off and dissolved in ethyl acetate and the solution is washed three times with water, dried with sodium sulfate and evaporated in vacuo. The residue is suspended in hexane and filtered. The title product is thus obtained with a melting point of 163-165°C.

### H1e) Methyl 2-[[[(1-methyl-2-phenyl-2-E-[(2-propynyl)oxyimino]ethylidene)amino]oxy]methyl]-α-(methoxymethylene)-phenylacetate

A solution of 5 g of 1-phenyl-1,2-propanedione 1-E-[(2-propynyl)oxime]-2-oxime in 24 ml of N,N-dimethylformamide is added dropwise to a suspension of 1.16 g of sodium hydride (about 55% in oil) in 45 ml of N,N-dimethylformamide at room temperature and the mixture is further stirred for 10 minutes. 6.5 g of methyl 2-(bromomethyl)-α-(methoxymethylene)phenylacetate in 24 ml of N,N-dimethylformamide are then added dropwise and the reaction mixture is further stirred at room temperature for 1 hour. Thereafter, the mixure is acidified with acetic acid and evaporated in vacuo. The residue is dissolved in ethyl acetate and the solution is washed three times with water and twice with saturated sodium chloride solution, dried with sodium sulfate and evaporated in vacuo. After recrystallization of the residue from hexane/ethyl acetate, the title compound is obtained with a melting point of 82-84°.

### Example H2: Methyl 2-[[[(1-methyl-2-(4-fluorophenyl)-2-E-[(2-propynyl)oxyimino]ethylidene)amino]oxy]methyl]-α-(methoxymethylene)-phenylacetate (compound 1.44)

The title compound with a melting point of 91-93° can be prepared in a manner analogous to that described in Example H1, starting from 1-(4-fluorophenyl)-2-propanone.

### Example H3: Methyl 2-[[[(1-methyl-2-(4-(3-trifluoromethylphenylmethoxy)-phenyl) 2-E-[(2-propynyl)oxyimino]ethylidene)amino]oxy]methyl]-α-(methoxymethylene)-phenylacetate (compound 1.240)

### H3a) 1-(4-Hydroxyphenyl)-2-propanone

A mixture of 82 g of 1-(4-methoxyphenyl)-2-propanone, 500 ml of acetic acid and 500 ml of aqueous hydrobromic acid is boiled under reflux for 2 hours and then evaporated in vacuo. The oily residue is extracted four times with 700 ml of hexane/ether (5:2) each time, the extract is evaporated and the residue is chromatographed over silica gel using hexane/ethyl acetate (3:1). 1-(4-Hydroxyphenyl)-2-propanone is thus obtained with a melting point of 40-41°.

### H3b) 1-[4-(3-Trifluoromethylphenylmethoxy)-phenyl]-2-propanone

A mixture of 5.8 g of 1-(4-hydroxyphenyl)-2-propanone, 61.6 g of potassium carbonate, 72.3 g of 1-(chloromethyl)-3-(trifluoromethyl)-benzene, and 1 g of potassium iodide in 800 ml of acetone is boiled under reflux for 5 hours. Thereafter, the reaction mixture is filtered and the filtrate is evaporated in vacuo. The residue is then dissolved in diethyl ether and the ethereal phase is washed three times with water, dried with sodium sulfate and evaporated. The 1-[4-(3-trifluoromethylphenylmethoxy)-phenyl]-2-propanone thus obtainable is employed in the next reaction stage without further purification.

### H3c) 1-[4-(3-Trifluoromethylphenylmethoxy)-phenyl]-1,2-propanedione 1-E-oxime

45 g of a 30% solution of sodium methanolate in methanol are slowly added dropwise to a solution of 59.6 g of 1-[4-(3-trifluoromethylphenylmethoxy)-phenyl]-2-propanone and 23.4 g of isopentyl nitrite in 300 ml of methanol such that the temperature does not exceed 20-25°. The reaction mixture is then further stirred at room temperature for 1 hour and thereafter evaporated in vacuo. The residue is dissolved in 600 ml of water and the solution is acidified with 10% hydrochloric acid. The precipitate which separates out is filtered off and dissolved in ethyl acetate and the organic phase is washed twice with water, dried with sodium sulfate and evaporated. After the crude product has been suspended in hexane and filtered, 1-[4-(3-trifluoromethylphenylmethoxy)-phenyl]-1,2-propanedione 1-E-oxime is obtained with a melting point of 134-136°.

### H3d) 1-[4-(3-Trifluoromethylphenylmethoxy)-phenyl]-1,2-propanedione 1-E-[(2-propynyl)oxime]

A mixture of 6 g of 1-[4-(3-trifluoromethylphenylmethoxy)-phenyl]-1,2-propanedione 1-E-oxime, 2.4 g of 1-bromo-2-propyne, 2.6 g of potassium carbonate and 0.5 g of potassium iodide in 40 ml of acetonitrile is boiled under reflux for 1 hour and then evaporated in vacuo and the residue is dissolved in ethyl acetate. The organic phase is washed twice with water and once with saturated sodium chloride solution, dried with sodium sulfate and evaporated. The crude 1-[4-(3-trifluoromethylphenylmethoxy)-phenyl]-1,2-propanedione 1-E-[(2-propynyl)oxime] thus obtainable is further processed without further purification.

### H3e) 1-[4-(3-Trifluoromethylphenylmethoxy)-phenyl]-1,2-propanedione 1-E-[(2-propynyl)oxime]-2-oxime

A mixture of 5.9 g of 1-[4-(3-trifluoromethylphenylmethoxy)-phenyl]-1,2-propanedione 1-E-[(2-propynyl)oxime], 2.3 g of hydroxylamine hydrochloride and 2.6 g of pyridine in 60 ml of ethanol is boiled under reflux for 1 hour and then concentrated in vacuo, and 200 ml of water are added to the residue. The product which has precipitated out is filtered off and dissolved in ethyl acetate, and the solution is washed twice with water and once with saturated sodium chloride solution, dried with sodium sulfate and evaporated in vacuo. The residue is suspended in hexane and filtered. 1-[4-(3-Trifluoromethylphenylmethoxy)-phenyl]-1,2-propanedione 1-E-[(2-propynyl)oxime]-2-oxime is thus obtained with a melting point of 114-115°.

### H3f) Methyl 2-[[[(1-methyl-2-(4-(3-trifluoromethylphenylmethoxy)-phenyl)-2-E-[(2-propynyl)oxyimino]ethylidene)amino]oxy]methyl]-α-(methoxymethylene)-phenylacetate

A solution of 5.5 g of 1-[4-(3-trifluoromethylphenylmethoxy)-phenyl]-1,2-propanedione 1-E-[(2-propynyl)oxime]-2-oxime in 25 ml of N,N-dimethylformamide is added dropwise to a suspension of 0.7 g of sodium hydride (about 55% in oil) in 25 ml of N,N-dimethylformamide and the mixture is further stirred at room temperature for 10 minutes. 4 g of methyl 2-(bromomethyl)-α-(methoxymethylene)-phenylacetate in 15 ml of N,N-dimethylformamide are then added dropwise and the reaction mixture is further stirred at room temperature for 1 hour. Thereafter, the mixture is acidified with acetic acid and evaporated in vacuo at 50°. The residue is dissolved in ethyl acetate and the solution is washed twice with water and once with saturated sodium chloride solution, dried with sodium sulfate and evaporated in vacuo. After purification by chromatography (silica gel, ethyl acetate/hexane 1:3), the title compound is obtained as a resin.

### Example H4: Methyl 2-[[[(1-methyl-2-(4-(4-chlorophenoxy)-phenyl)-2-E-[(2-ethyl)oxyimino]ethylidene)amino]oxy]methyl]-α-(methoxymethylene)-phenylacetate (compound 1.366)

### H4a) 1-[4-(4-Chlorophenoxy)-phenyl]-1,2-propanedione 1-E-oxime

16.7 g of a 30% solution of sodium methylate in methanol are added dropwise to a solution of 22.5 g of 1-[4-(4-chlorophenoxy)-phenyl]-2-propanone and 10.3 g of isopentyl nitrite in 120 ml of methanol at 20-25°, while cooling. The reaction mixture is then further stirred at room temperature for 1 hour. After the solution has been concentrated in vacuo, the residue is dissolved in 300 ml of water and the solution is acidified with 10% hydrochloric acid, the product which precipitates out is filtered off and dissolved in ethyl acetate and the organic phase is washed twice with water, dried with sodium sulfate and evaporated in vacuo. The residue is stirred up in hexane and filtered. The title product is thus obtained with a melting point of 154-155°C.

### H4b) 1-[4-(4-Chlorophenoxy)-phenyl]-1,2-propanedione 1-E-[(2-ethyl)oxime]

A mixture of 6 g of ) 1-[4-(4-Chlorophenoxy)-phenyl]-1,2-propanedione 1-E-oxime, 3.3 g of ethyl bromide, 3.5 g of potassium carbonate and 0.5 g of potassium iodide in 30 ml of acetonitrile is stirred at 50° for 2 hours, the solvent is then distilled off in vacuo and the residue is dissolved again in ethyl acetate. The organic phase is washed in each case twice with water and saturated sodium chloride solution, dried with sodium sulfate and evaporated in vacuo. After recrystallization of the residue from hexane, the title product is obtained with a melting point of 77-78°C.

### H4c) 1-[4-(4-Chlorophenoxy)-phenyl]-1,2-propanedione 1-E-[(2-ethyl)oxime]-2-oxime

A mixture of 5.5 g of 1-[4-(4-chlorophenoxy)-phenyl]-1,2-propanedione 1-E-[(2-ethyl)oxime], 2.4 g of hydroxylamine hydrochloride and 2.7 g of pyridine in 50 ml of ethanol is boiled under reflux for 1 hour and then concentrated in vacuo, and 800 ml of water are added to the residue. The product which has precipitated out is filtered off and dissolved in ethyl acetate and the solution is washed three times with water, dried with sodium sulfate and evaporated in vacuo. The residue is suspended in hexane and filtered. The title product is thus obtained in a pure form with a melting point of 176-177°C.

### H4d) Methyl 2-[[[(1-methyl-2-(4-(4-chlorophenoxy)-phenyl)-2-E-[(2-ethyl)oxyimino]ethylidene)amino]oxy]methyl]-α-(methoxymethylene)-phenylacetate.

A solution of 4.7 g of 1-[4-(4-chlorophenoxy)-phenyl]-1,2-propanedione 1-E-[(2-propynyl)-oxime]-2-oxime in 25 ml of N,N-dimethylformamide is added dropwise to a suspension of 0.65 g of sodium hydride (about 55% in oil) in 20 ml of N,N-dimethylformamide and the mixture is further stirred at room temperature for 10 minutes. 4 g of methyl 2-(bromomethyl)-α-(methoxymethylene)-phenylacetate in 15 ml of N,N-dimethyiformamide are then added dropwise and the reaction mixture is further stirred at room temperature for 1 hour. Thereafter, the mixture is acidified with acetic acid and evaporated in vacuo at 50°. The residue is dissolved in ethyl acetate and the solution is washed twice with water and once with saturated sodium chloride solution, dried with sodium sulfate and evaporated in vacuo. After purification by flash chromatography (silica gel, ethyl acetate/hexane 1:3), the title compound is obtained with a melting point of 87-89°C.

### Example H5: Methyl 2-[[[(1-methyl-2-(4-(4-chlorophenoxy)-phenyl)-2-E-[(2-ethyl)oxyimino]ethylidene)amino]oxy]methyl]-α-(methoxyimino)-phenylacetate (compound 2.366)

The title compound with a melting point of 90 to 93°C is obtained in a manner analogous to that described in Example H4 from 1-[4-(4-chlorophenoxy)-phenyl]-1,2-propanedione 1-E-[(2-propynyl)oxime]-2-oxime and methyl 2-(bromomethyl)-α-(methoxyimino)-phenylacetate.

### Example H6: 2-[[[(1-Methyl-2-(4-(4-chlorophenoxy)-phenyl)-2-E-[(2-ethyl)oxyimino]ethylidene)amino]oxy]methyl]-α-(methoxyimino)-phenylacetic acid methylamide (compound 3.366)

13,3 g of methyl 2-[[[(1-methyl-2-(4-(4-chlorophenoxy)-phenyl)-2-E-[(2-ethyl)oxyimino]ethyliene)amino]oxy]methyl]-α-(methoxyimino)-phenylacetate are left to stand together with 80 ml of dimethylformamide and 9.2 ml of an 8 molar solution of methylamine in ethanol at room temperature for two days. The mixture is concentrated at 50°C, n-hexane is added and the mixture is cooled to room temperature and filtered. The residue is dried under a high vacuum. The title compound is obtained with a melting point of 126-129°C.

### Example H7: The other compounds listed in Tables 1 to 3 can also be prepared in a manner analogous to that described in Examples H1 to H6. In the "physical data" column of the tables, the temperatures stated in each case designate the melting point of the compound in question. c.propyl is cyclopropyl.

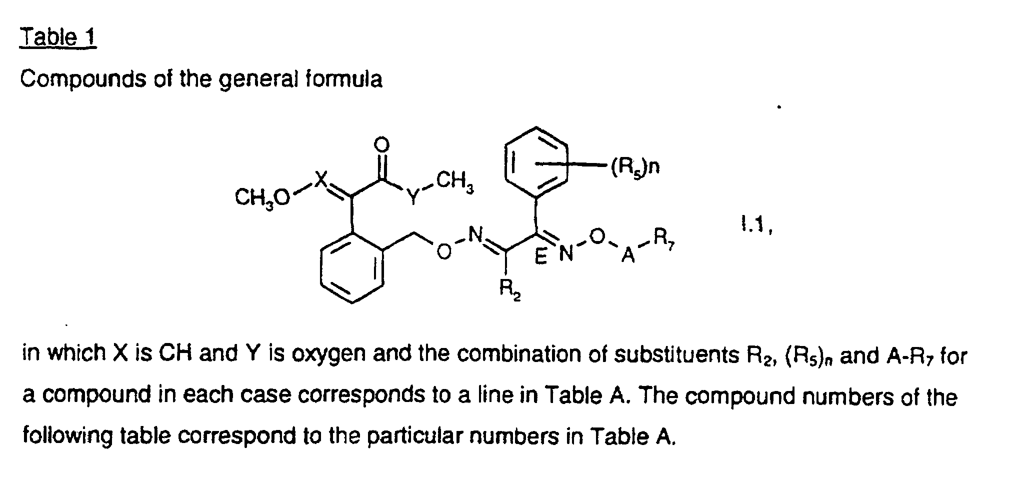

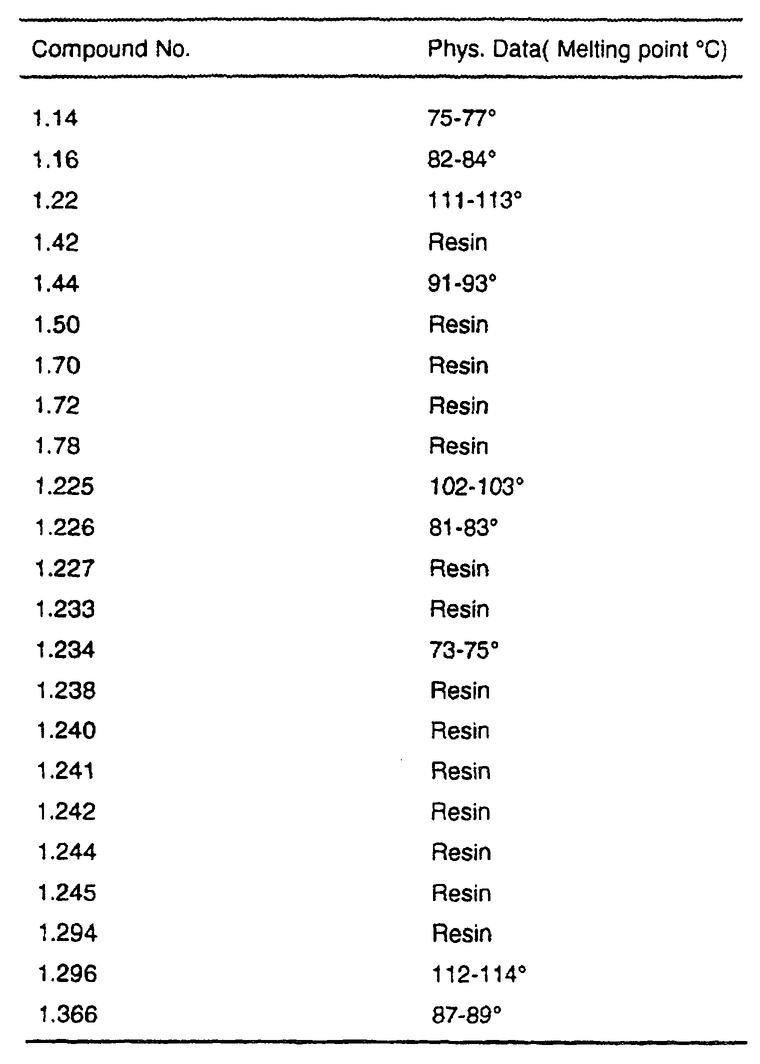

**Table 2**

| | |
|---|---|
| Compounds of the general formula I. 1, in which X is nitrogen and Y is oxygen and the combination of substituents R₂, (R₅)ₙ and A-R₇ for a compound in each case corresponds to a line in Table A. | |

| Compound No. | Melting point (°C) |
|---|---|
| 2.198 | 75-77 |
| 2.254 | 80-82 |
| 2.309 | 106-108 |
| 2.310 | 102-104 |
| 2.366 | 90-93 |

**Table 3**

| | |
|---|---|
| Compounds of the general formula I.1, in which X is nitrogen and Y is NH and the combination of substituents R₂, (R₅)ₙ and A-R₇ for a compound in each case corresponds to a line in Table A. | |

| Compound No. | Melting point (°C) |
|---|---|
| 3.198 | 75-77 |
| 3.254 | 112-114 |
| 3.309 | 89-91 |
| 3.310 | 88-90 |
| 3.366 | 126-129 |

**Table A**

| Compound No. | R₂ | (R₅)ₙ | A-R₇ |
|---|---|---|---|
| 1 | CH₃ | H | CH₃ |
| 2 | CH₃ | H | C₂H₅ |
| 3 | CH₃ | H | n-C₃H₇ |
| 4 | CH₃ | H | i-C₃H₇ |
| 5 | CH₃ | H | n-C₄H₉ |
| 6 | CH₃ | H | n-C₆H₁₃ |
| 7 | CH₃ | H | CH₂F |
| 8 | CH₃ | H | CHF₂ |
| 9 | CH₃ | H | CH₂CF₃ |
| 10 | CH₃ | H | CH₂CH=CH₂ |
| 11 | CH₃ | H | CH₂CH=CHCH₃ |
| 12 | CH₃ | H | CH₂CH=C(CH₃)₂ |
| 13 | CH₃ | H | CH₂CH=CHCl |
| 14 | CH₃ | H | CH₂CH=CCl₂ |
| 15 | CH₃ | H | CH₂C(CH₃)=CH₂ |
| 16 | CH₃ | H | CH₂C≡CH |
| 17 | CH₃ | H | CH₂Si(CH₃)₃ |
| 18 | CH₃ | H | CH₂-c.propyl-2,2-Cl₂ |
| 19 | CH₃ | H | CH₂CN |
| 20 | CH₃ | H | CH₂COOC₂H₅ |
| 21 | CH₃ | H | CH(CH₃)COOC₂H₅ |
| 22 | CH₃ | H | CH₂C₆H₄-3-CF₃ |
| 23 | CH₃ | H | CH₂C₆H₄-4-F |
| 24 | CH₃ | H | CH₂C₆H₄-3-F |
| 25 | CH₃ | H | CH₂C₆H₄-2-F |
| 26 | CH₃ | H | C(=O)OC₂H₅ |
| 27 | CH₃ | H | C(=O)NHCH₃ |
| 28 | CH₃ | H | C(=O)C(=O)OC₂H₅ |
| 29 | CH₃ | 4-F | CH₃ |
| 30 | CH₃ | 4-F | C₂H₅ |
| 31 | CH₃ | 4-F | n-C₃H₇ |
| 32 | CH₃ | 4-F | i-C₃H₇ |
| 33 | CH₃ | 4-F | n-C₄H₉ |
| 34 | CH₃ | 4-F | n-C₆H₁₃ |
| 35 | CH₃ | 4-F | CH₂F |
| 36 | CH₃ | 4-F | CHF₂ |
| 37 | CH₃ | 4-F | CH₂CF₃ |
| 38 | CH₃ | 4-F | CH₂CH=CH₂ |
| 39 | CH₃ | 4-F | CH₂CH=CHCH₃ |
| 40 | CH₃ | 4-F | CH₂CH=C(CH₃)₂ |
| 41 | CH₃ | 4-F | CH₂CH=CHCl |
| 42 | CH₃ | 4-F | CH₂CH=CCl₂ |
| 43 | CH₃ | 4-F | CH₂C(CH₃)=CH₂ |
| 44 | CH₃ | 4-F | CH₂C≡CH |
| 45 | CH₃ | 4-F | CH₂Si(CH₃)₃ |
| 46 | CH₃ | 4-F | CH₂-c.propyl-2,2-Cl₂ |
| 47 | CH₃ | 4-F | CH₂CN |
| 48 | CH₃ | 4-F | CH₂COOC₂H₅ |
| 49 | CH₃ | 4-F | CH(CH₃)COOC₂H₅ |
| 50 | CH₃ | 4-F | CH₂C₆H₄-3-CF₃ |
| 51 | CH₃ | 4-F | CH₂C₆H₄-4-F |
| 52 | CH₃ | 4-F | CH₂C₆H₄-3-F |
| 53 | CH₃ | 4-F | CH₂C₆H₄-2-F |
| 54 | CH₃ | 4-F | C(=O)OC₂H₅ |
| 55 | CH₃ | 4-F | C(=O)NHCH₃ |
| 56 | CH₃ | 4-F | C(=O)C(=O)OC₂H₅ |
| 57 | CH₃ | 4-OCH₃ | CH₃ |
| 58 | CH₃ | 4-OCH₃ | C₂H₅ |
| 59 | CH₃ | 4-OCH₃ | n-C₃H₇ |
| 60 | CH₃ | 4-OCH₃ | i-C₃H₇ |
| 61 | CH₃ | 4-OCH₃ | n-C₄H₉ |
| 62 | CH₃ | 4-OCH₃ | n-C₆H₁₃ |
| 63 | CH₃ | 4-OCH₃ | CH₂F |
| 64 | CH₃ | 4-OCH₃ | CHF₂ |
| 65 | CH₃ | 4-OCH₃ | CH₂CF₃ |
| 66 | CH₃ | 4-OCH₃ | CH₂CH=CH₂ |
| 67 | CH₃ | 4-OCH₃ | CH₂CH=CHCH₃ |
| 68 | CH₃ | 4-OCH₃ | CH₂CH=C(CH₃)₂ |
| 69 | CH₃ | 4-OCH₃ | CH₂CH=CHCl |
| 70 | CH₃ | 4-OCH₃ | CH₂CH=CCl₂ |
| 71 | CH₃ | 4-OCH₃ | CH₂C(CH)=CH₂ |
| 72 | CH₃ | 4-OCH₃ | CH₂C≡CH |
| 73 | CH₃ | 4-OCH₃ | CH₂Si(CH₃)₃ |
| 74 | CH₃ | 4-OCH₃ | CH₂-c.propyl-2,2-Cl₂ |
| 75 | CH₃ | 4-OCH₃ | CH₂CN |
| 76 | CH₃ | 4-OCH₃ | CH₂COOC₂H₅ |
| 77 | CH₃ | 4-OCH₃ | CH(CH₃)COOC₂H₅ |
| 78 | CH₃ | 4-OCH₃ | CH₂C₆H₄-3-CF₃ |
| 79 | CH₃ | 4-OCH₃ | CH₂C₆H₄-4-F |
| 80 | CH₃ | 4-OCH₃ | CH₂C₆H₄-3-F |
| 81 | CH₃ | 4-OCH₃ | CH₂C₆H₄-2-F |
| 82 | CH₃ | 4-OCH₃ | C(=O)OC₂H₅ |
| 83 | CH₃ | 4-OCH₃ | C(=O)NHCH₃ |
| 84 | CH₃ | 4-OCH₃ | C(=O)C(=O)OC₂H₅ |
| 85 | CH₃ | 4-OC₂H₅ | CH₃ |
| 86 | CH₃ | 4-OC₂H₅ | C₂H₅ |
| 87 | CH₃ | 4-OC₂H₅ | n-C₃H₇ |
| 88 | CH₃ | 4-OC₂H₅ | i-C₃H₇ |
| 89 | CH₃ | 4-OC₂H₅ | n-C₄H₉ |
| 90 | CH₃ | 4-OC₂H₅ | n-C₆H₁₃ |
| 91 | CH₃ | 4-OC₂H₅ | CH₂F |
| 92 | CH₃ | 4-OC₂H₅ | CHF₂ |
| 93 | CH₃ | 4-OC₂H₅ | CH₂CF₃ |
| 94 | CH₃ | 4-OC₂H₅ | CH₂CH=CH₂ |
| 95 | CH₃ | 4-OC₂H₅ | CH₂CH=CHCH₃ |
| 96 | CH₃ | 4-OC₂H₅ | CH₂CH=C(CH₃)₂ |
| 97 | CH₃ | 4-OC₂H₅ | CH₂CH=CHCl |
| 98 | CH₃ | 4-OC₂H₅ | CH₂CH=CCl₂ |
| 99 | CH₃ | 4-OC₂H₅ | CH₂C(CH₃)=CH₂ |
| 100 | CH₃ | 4-OC₂H₅ | CH₂C≡CH |
| 101 | CH₃ | 4-OC₂H₅ | CH₂Si(CH₃)₃ |
| 102 | CH₃ | 4-OC₂H₅ | CH₂-c.propyl-2,2-Cl₂ |
| 103 | CH₃ | 4-OC₂H₅ | CH₂CN |
| 104 | CH₃ | 4-OC₂H₅ | CH₂COOC₂H₅ |
| 105 | CH₃ | 4-OC₂H₅ | CH(CH₃)COOC₂H₅ |
| 106 | CH₃ | 4-OC₂H₅ | CH₂C₆H₄-3-CF₃ |
| 107 | CH₃ | 4-OC₂H₅ | CH₂C₆H₄-4-F |
| 108 | CH₃ | 4-OC₂H₅ | CH₂C₆H₄-3-F |
| 109 | CH₃ | 4-OC₂H₅ | CH₂C₆H₄-2-F |
| 110 | CH₃ | 4-OC₂H₅ | C(=O)OC₂H₅ |
| 111 | CH₃ | 4-OC₂H₅ | C(=O)NHCH₃ |
| 112 | CH₃ | 4-OC₂H₅ | C(=O)C(=O)OC₂H₅ |
| 113 | CH₃ | 4-O-n-C₃H₇ | CH₃ |
| 114 | CH₃ | 4-O-n-C₃H₇ | C₂H₅ |
| 115 | CH₃ | 4-O-n-C₃H₇ | n-C₃H₇ |
| 116 | CH₃ | 4-O-n-C₃H₇ | i-C₃H₇ |
| 117 | CH₃ | 4-O-n-C₃H₇ | n-C₄H₉ |
| 118 | CH₃ | 4-O-n-C₃H₇ | n-C₆H₁₃ |
| 119 | CH₃ | 4-O-n-C₃H₇ | CH₂F |
| 120 | CH₃ | 4-O-n-C₃H₇ | CHF₂ |
| 121 | CH₃ | 4-O-n-C₃H₇ | CH₂CF₃ |
| 122 | CH₃ | 4-O-n-C₃H₇ | CH₂CH=CH₂ |
| 123 | CH₃ | 4-O-n-C₃H₇ | CH₂CH=CHCH₃ |
| 124 | CH₃ | 4-O-n-C₃H₇ | CH₂CH=C(CH₃)₂ |
| 125 | CH₃ | 4-O-n-C₃H₇ | CH₂CH=CHCl |
| 126 | CH₃ | 4-O-n-C₃H₇ | CH₂CH=CCl₂ |
| 127 | CH₃ | 4-O-n-C₃H₇ | CH₂C(CH₃)=CH₂ |
| 128 | CH₃ | 4-O-n-C₃H₇ | CH₂C≡CH |
| 129 | CH₃ | 4-O-n-C₃H₇ | CH₂Si(CH₃)₃ |
| 130 | CH₃ | 4-O-n-C₃H₇ | CH₂-c.propyl-2,2-Cl₂ |
| 131 | CH₃ | 4-O-n-C₃H₇ | CH₂CN |
| 132 | CH₃ | 4-O-n-C₃H₇ | CH₂COOC₂H₅ |
| 133 | CH₃ | 4-O-n-C₃H₇ | CH(CH₃)COOC₂H₅ |
| 134 | CH₃ | 4-O-n-C₃H₇ | CH₂C₆H₄-3-CF₃ |
| 135 | CH₃ | 4-O-n-C₃H₇ | CH₂C₆H₄-4-F |
| 136 | CH₃ | 4-O-n-C₃H₇ | CH₂C₆H₄-3-F |
| 137 | CH₃ | 4-O-n-C₃H₇ | CH₂C₆H₄-2-F |
| 138 | CH₃ | 4-O-n-C₃H₇ | C(=O)OC₂H₅ |
| 139 | CH₃ | 4-O-n-C₃H₇ | C(=O)NHCH₃ |
| 140 | CH₃ | 4-O-n-C₃H₇ | C(=O)C(=O)OC₂H₅ |
| 141 | CH₃ | 2-CH₃ | CH₃ |
| 142 | CH₃ | 2-CH₃ | C₂H₅ |
| 143 | CH₃ | 2-CH₃ | n-C₃H₇ |
| 144 | CH₃ | 2-CH₃ | i-C₃H₇ |
| 145 | CH₃ | 2-CH₃ | n-C₄H₉ |
| 146 | CH₃ | 2-CH₃ | n-C₆H₁₃ |
| 147 | CH₃ | 2-CH₃ | CH₂F |
| 148 | CH₃ | 2-CH₃ | CHF₂ |
| 149 | CH₃ | 2-CH₃ | CH₂CF₃ |
| 150 | CH₃ | 2-CH₃ | CH₂CH=CH₂ |
| 151 | CH₃ | 2-CH₃ | CH₂CH=CHCH₃ |
| 152 | CH₃ | 2-CH₃ | CH₂CH=C(CH₃)₂ |
| 153 | CH₃ | 2-CH₃ | CH₂CH=CHCl |
| 154 | CH₃ | 2-CH₃ | CH₂CH=CCl₂ |
| 155 | CH₃ | 2-CH₃ | CH₂C(CH₃)=CH₂ |
| 156 | CH₃ | 2-CH₃ | CH₂C≡CH |
| 157 | CH₃ | 2-CH₃ | CH₂Si(CH₃)₃ |
| 158 | CH₃ | 2-CH₃ | CH₂-c.propyl-2,2-Cl₂ |
| 159 | CH₃ | 2-CH₃ | CH₂CN |
| 160 | CH₃ | 2-CH₃ | CH₂COOC₂H₅ |
| 161 | CH₃ | 2-CH₃ | CH(CH₃)COOC₂H₅ |
| 162 | CH₃ | 2-CH₃ | CH₂C₆H₄-3-CF₃ |
| 163 | CH₃ | 2-CH₃ | CH₂C₆H₄-4-F |
| 164 | CH₃ | 2-CH₃ | CH₂C₆H₄-3-F |
| 165 | CH₃ | 2-CH₃ | CH₂C₆H₄-2-F |
| 166 | CH₃ | 2-CH₃ | C(=O)OC₂H₅ |
| 167 | CH₃ | 2-CH₃ | C(=O)NHCH₃ |
| 168 | CH₃ | 2-CH₃ | C(=O)C(=O)OC₂H₅ |
| 169 | CH₃ | 4-OCH₂Si(CH₃)₃ | CH₃ |
| 170 | CH₃ | 4-OCH₂Si(CH₃)₃ | C₂H₅ |
| 171 | CH₃ | 4-OCH₂Si(CH₃)₃ | n-C₃H₇ |
| 172 | CH₃ | 4-OCH₂Si(CH₃)₃ | i-C₃H₇ |
| 173 | CH₃ | 4-OCH₂Si(CH₃)₃ | n-C₄H₉ |
| 174 | CH₃ | 4-OCH₂Si(CH₃)₃ | n-C₆H₁₃ |
| 175 | CH₃ | 4-OCH₂Si(CH₃)₃ | CH₂F |
| 176 | CH₃ | 4-OCH₂Si(CH₃)₃ | CHF₂ |
| 177 | CH₃ | 4-OCH₂Si(CH₃)₃ | CH₂CF₃ |
| 178 | CH₃ | 4-OCH₂Si(CH₃)₃ | CH₂CH=CH₂ |
| 179 | CH₃ | 4-OCH₂Si(CH₃)₃ | CH₂CH=CHCH₃ |
| 180 | CH₃ | 4-OCH₂Si(CH₃)₃ | CH₂CH=C(CH₃)₂ |
| 181 | CH₃ | 4-OCH₂Si(CH₃)₃ | CH₂CH=CHCl |
| 182 | CH₃ | 4-OCH₂Si(CH₃)₃ | CH₂CH=CCl₂ |
| 183 | CH₃ | 4-OCH₂Si(CH₃)₃ | CH₂C(CH₃)=CH₂ |
| 184 | CH₃ | 4-OCH₂Si(CH₃)₃ | CH₂C≡CH |
| 185 | CH₃ | 4-OCH₂Si(CH3)₃ | CH₂Si(CH₃)₃ |
| 186 | CH₃ | 4-OCH₂Si(CH₃)₃ | CH₂-c.propyl-2,2-Cl₂ |
| 187 | CH₃ | 4-OCH₂Si(CH3)₃ | CH₂CN |
| 188 | CH₃ | 4-OCH₂Si(CH₃)₃ | CH₂COOC₂H₅ |
| 189 | CH₃ | 4-OCH₂Si(CH₃)₃ | CH(CH₃)COOC₂H₅ |
| 190 | CH₃ | 4-OCH₂Si(CH₃)₃ | CH₂C₆H₄-3-CF₃ |
| 191 | CH₃ | 4-OCH₂Si(CH3)₃ | CH₂C₆H₄-4-F |
| 192 | CH₃ | 4-OCH₂Si(CH₃)₃ | CH₂C₆H₄-3-F |
| 193 | CH₃ | 4-OCH₂Si(CH₃)₃ | CH₂C₆H₄-2-F |
| 194 | CH₃ | 4-OCH₂Si(CH₃)₃ | C(=O)OC₂H₅ |
| 195 | CH₃ | 4-OCH₂Si(CH₃)₃ | C(=O)NHCH₃ |
| 196 | CH₃ | 4-OCH₂Si(CH₃)₃ | C(=O)C(=O)OC₂H₅ |
| 197 | CH₃ | 4-OCH₂C₆H₄-4-CF₃ | CH₃ |
| 198 | CH₃ | 4-OCH₂C₆H₄-4-CF₃ | C₂H₅ |
| 199 | CH₃ | 4-OCH₂C₆H₄-4-CF₃ | n-C₃H₇ |
| 200 | CH₃ | 4-OCH₂C₆H₄-4-CF₃ | i-C₃H₇ |
| 201 | CH₃ | 4-OCH₂C₆H₄-4-CF₃ | n-C₄H₉ |
| 202 | CH₃ | 4-OCH₂C₆H₄-4-CF₃ | n-C₆H₁₃ |
| 203 | CH₃ | 4-OCH₂C₆H₄-4-CF₃ | CH₂F |
| 204 | CH₃ | 4-OCH₂C₆H₄-4-CF₃ | CHF₂ |
| 205 | CH₃ | 4-OCH₂C₆H₄-4-CF₃ | CH₂CF₃ |
| 206 | CH₃ | 4-OCH₂C₆H₄-4-CF₃ | CH₂CH=CH₂ |
| 207 | CH₃ | 4-OCH₂C₆H₄-4-CF₃ | CH₂CH=CHCH₃ |
| 208 | CH₃ | 4-OCH₂C₆H₄-4-CF₃ | CH₂CH=C(CH₃)₂ |
| 209 | CH₃ | 4-OCH₂C₆H₄-4-CF₃ | CH₂CH=CHCl |
| 210 | CH₃ | 4-OCH₂C₆H₄-4-CF₃ | CH₂CH=CCl₂ |
| 211 | CH₃ | 4-OCH₂C₆H₄-4-CF₃ | CH₂C(CH₃)=CH₂ |
| 212 | CH₃ | 4-OCH₂C₆H₄-4-CF₃ | CH₂C≡CH |
| 213 | CH₃ | 4-OCH₂C₆H₄-4-CF₃ | CH₂Si(CH₃)₃ |
| 214 | CH₃ | 4-OCH₂C₆H₄-4-CF₃ | CH₂-c.propyl-2,2-Cl₂ |
| 215 | CH₃ | 4-OCH₂C₆H₄-4-CF₃ | CH₂CN |
| 216 | CH₃ | 4-OCH₂C₆H₄-4-CF₃ | CH₂COOC₂H₅ |
| 217 | CH₃ | 4-OCH₂C₆H₄-4-CF₃ | CH(CH₃)COOC₂H₅ |
| 218 | CH₃ | 4-OCH₂C₆H₄-4-CF₃ | CH₂C₆H₄-3-CF₃ |
| 219 | CH₃ | 4-OCH₂C₆H₄-4-CF₃ | CH₂C₆H₄-4-F |
| 220 | CH₃ | 4-OCH₂C₆H₄-4-CF₃ | CH₂C₆H₄-3-F |
| 221 | CH₃ | 4-OCH₂C₆H₄-4-CF₃ | CH₂C₆H₄-2-F |
| 222 | CH₃ | 4-OCH₂C₆H₄-4-CF₃ | C(=O)OC₂H₅ |
| 223 | CH₃ | 4-OCH₂C₆H₄-4-CF₃ | C(=O)NHCH₃ |
| 224 | CH₃ | 4-OCH₂C₆H₄-4-CF₃ | C(=O)C(=O)OC₂H₅ |
| 225 | CH₃ | 4-OCH₂C₆H₄-3-CF₃ | CH₃ |
| 226 | CH₃ | 4-OCH₂C₆H₄-3-CF₃ | C₂H₅ |
| 227 | CH₃ | 4-OCH₂C₆H₄-3-CF₃ | n-C₃H₇ |
| 228 | CH₃ | 4-OCH₂C₆H₄-3-CF₃ | i-C₃H₇ |
| 229 | CH₃ | 4-OCH₂C₆H₄-3-CF₃ | n-C₄H₉ |
| 230 | CH₃ | 4-OCH₂C₆H₄-3-CF₃ | n-C₆H₁₃ |
| 231 | CH₃ | 4-OCH₂C₆H₄-3-CF₃ | CH₂F |
| 232 | CH₃ | 4-OCH₂C₆H₄-3-CF₃ | CHF₂ |
| 233 | CH₃ | 4-OCH₂C₆H₄-3-CF₃ | CH₂CF₃ |
| 234 | CH₃ | 4-OCH₂C₆H₄-3-CF₃ | CH₂CH=CH₂ |
| 235 | CH₃ | 4-OCH₂C₆H₄-3-CF₃ | CH₂CH=CHCH₃ |
| 236 | CH₃ | 4-OCH₂C₆H₄-3-CF₃ | CH₂CH=C(CH₃)₂ |
| 237 | CH₃ | 4-OCH₂C₆H₄-3-CF₃ | CH₂CH=CHCl |
| 238 | CH₃ | 4-OCH₂C₆H₄-3-CF₃ | CH₂CH=CCl₂ |
| 239 | CH₃ | 4-OCH₂C₆H₄-3-CF₃ | CH₂C(CH₃)=CH₂ |
| 240 | CH₃ | 4-OCH₂C₆H₄-3-CF₃ | CH₂C≡CH |
| 241 | CH₃ | 4-OCH₂C₆H₄-3-CF₃ | CH₂Si(CH₃)₃ |
| 242 | CH₃ | 4-OCH₂C₆H₄-3-CF₃ | CH₂-c.propyl-2,2-Cl₂ |
| 243 | CH₃ | 4-OCH₂C₆H₄-3-CF₃ | CH₂CN |
| 244 | CH₃ | 4-OCH₂C₆H₄-3-CF₃ | CH₂COOC₂H₅ |
| 245 | CH₃ | 4-OCH₂C₆H₄-3-CF₃ | CH(CH₃)COOC₂H₅ |
| 246 | CH₃ | 4-OCH₂C₆H₄-3-CF₃ | CH₂C₆H₄-3-CF₃ |
| 247 | CH₃ | 4-OCH₂C₆H₄-3-CF₃ | CH₂C₆H₄-4-F |
| 248 | CH₃ | 4-OCH₂C₆H₄-3-CF₃ | CH₂C₆H₄-3-F |
| 249 | CH₃ | 4-OCH₂C₆H₄-3-CF₃ | CH₂C₆H₄-2-F |
| 250 | CH₃ | 4-OCH₂C₆H₄-3-CF₃ | C(=O)OC₂H₅ |
| 251 | CH₃ | 4-OCH₂C₆H₄-3-CF₃ | C(=O)NHCH₃ |
| 252 | CH₃ | 4-OCH₂C₆H₄-3-CF₃ | C(=O)C(=O)OC₂H₅ |
| 253 | CH₃ | 4-OCH₂C₆H₄-2-CF₃ | CH₃ |
| 254 | CH₃ | 4-OCH₂C₆H₄-2-CF₃ | C₂H₅ |
| 255 | CH₃ | 4-OCH₂C₆H₄-2-CF₃ | n-C₃H₇ |
| 256 | CH₃ | 4-OCH₂C₆H₄-2-CF₃ | i-C₃H₇ |
| 257 | CH₃ | 4-OCH₂C₆H₄-2-CF₃ | n-C₄H₉ |
| 258 | CH₃ | 4-OCH₂C₆H₄-2-CF₃ | n-C₆H₁₃ |
| 259 | CH₃ | 4-OCH₂C₆H₄-2-CF₃ | CH₂F |
| 260 | CH₃ | 4-OCH₂C₆H₄-2-CF₃ | CHF₂ |
| 261 | CH₃ | 4-OCH₂C₆H₄-2-CF₃ | CH₂CF₃ |
| 262 | CH₃ | 4-OCH₂C₆H₄-2-CF₃ | CH₂CH=CH₂ |
| 263 | CH₃ | 4-OCH₂C₆H₄-2-CF₃ | CH₂CH=CHCH₃ |
| 264 | CH₃ | 4-OCH₂C₆H₄-2-CF₃ | CH₂CH=C(CH₃)₂ |
| 265 | CH₃ | 4-OCH₂C₆H₄-2-CF₃ | CH₂CH=CHCl |
| 266 | CH₃ | 4-OCH₂C₆H₄-2-CF₃ | CH₂CH=CCl₂ |
| 267 | CH₃ | 4-OCH₂C₆H₄-2-CF₃ | CH₂C(CH₃)=CH₂ |
| 268 | CH₃ | 4-OCH₂C₆H₄-2-CF₃ | CH₂C≡CH |
| 269 | CH₃ | 4-OCH₂C₆H₄-2-CF₃ | CH₂Si(CH₃)₃ |
| 270 | CH₃ | 4-OCH₂C₆H₄-2-CF₃ | CH₂-c.propyl-2,2-Cl₂ |
| 271 | CH₃ | 4-OCH₂C₆H₄-2-CF₃ | CH₂CN |
| 272 | CH₃ | 4-OCH₂C₆H₄-2-CF₃ | CH₂COOC₂H₅ |
| 273 | CH₃ | 4-OCH₂C₆H₄-2-CF₃ | CH(CH₃)COOC₂H₅ |
| 274 | CH₃ | 4-OCH₂C₆H₄-2-CF₃ | CH₂C₆H₄-3-CF₃ |
| 275 | CH₃ | 4-OCH₂C₆H₄-2-CF₃ | CH₂C₆H₄-4-F |
| 276 | CH₃ | 4-OCH₂C₆H₄-2-CF₃ | CH₂C₆H₄-3-F |
| 277 | CH₃ | 4-OCH₂C₆H₄-2-CF₃ | CH₂C₆H₄-2-F |
| 278 | CH₃ | 4-OCH₂C₆H₄-2-CF₃ | C(=O)OC₂H₅ |
| 279 | CH₃ | 4-OCH₂C₆H₄-2-CF₃ | C(=O)NHCH₃ |
| 280 | CH₃ | 4-OCH₂C₆H₄-2-CF₃ | C(=O)C(=O)OC₂H₅ |
| 281 | CH₃ | 4-OCH₂C₆H₄-4-F | CH₃ |
| 282 | CH₃ | 4-OCH₂C₆H₄-4-F | C₂H₅ |
| 283 | CH₃ | 4-OCH₂C₆H₄-4-F | n-C₃H₇ |
| 284 | CH₃ | 4-OCH₂C₆H₄-4-F | i-C₃H₇ |
| 285 | CH₃ | 4-OCH₂C₆H₄-4-F | n-C₄H₉ |
| 286 | CH₃ | 4-OCH₂C₆H₄-4-F | n-C₆H₁₃ |
| 287 | CH₃ | 4-OCH₂C₆H₄-4-F | CH₂F |
| 288 | CH₃ | 4-OCH₂C₆H₄-4-F | CHF₂ |
| 289 | CH₃ | 4-OCH₂C₆H₄-4-F | CH₂CF₃ |
| 290 | CH₃ | 4-OCH₂C₆H₄-4-F | CH₂CH=CH₂ |
| 291 | CH₃ | 4-OCH₂C₆H₄-4-F | CH₂CH=CHCH₃ |
| 292 | CH₃ | 4-OCH₂C₆H₄-4-F | CH₂CH=C(CH₃)₂ |
| 293 | CH₃ | 4-OCH₂C₆H₄-4-F | CH₂CH=CHCl |
| 294 | CH₃ | 4-OCH₂C₆H₄-4-F | CH₂CH=CCl₂ |
| 295 | CH₃ | 4-OCH₂C₆H₄-4-F | CH₂C(CH₃)=CH₂ |
| 296 | CH₃ | 4-OCH₂C₆H₄-4-F | CH₂C≡CH |
| 297 | CH₃ | 4-OCH₂C₆H₄-4-F | CH₂Si(CH₃)₃ |
| 298 | CH₃ | 4-OCH₂C₆H₄-4-F | CH₂-c.propyl-2,2-Cl₂ |
| 299 | CH₃ | 4-OCH₂C₆H₄-4-F | CH₂CN |
| 300 | CH₃ | 4-OCH₂C₆H₄-4-F | CH₂COOC₂H₅ |
| 301 | CH₃ | 4-OCH₂C₆H₄-4-F | CH(CH₃)COOC₂H₅ |
| 302 | CH₃ | 4-OCH₂C₆H₄-4-F | CH₂C₆H₄-3-CF₃ |
| 303 | CH₃ | 4-OCH₂C₆H₄-4-F | CH₂C₆H₄-4-F |
| 304 | CH₃ | 4-OCH₂C₆H₄-4-F | CH₂C₆H₄-3-F |
| 305 | CH₃ | 4-OCH₂C₆H₄-4-F | CH₂C₆H₄-2-F |
| 306 | CH₃ | 4-OCH₂C₆H₄-4-F | C(=O)OC₂H₅ |
| 307 | CH₃ | 4-OCH₂C₆H₄-4-F | C(=O)NHCH₃ |
| 308 | CH₃ | 4-OCH₂C₆H₄-4-F | C(=O)C(=O)OC₂H₅ |
| 309 | CH₃ | 4-OC₆H₄-3-CF₃ | CH₃ |
| 310 | CH₃ | 4-OC₆H₄-3-CF₃ | C₂H₅ |
| 311 | CH₃ | 4-OC₆H₄-3-CF₃ | n-C₃H₇ |
| 312 | CH₃ | 4-OC₆H₄-3-CF₃ | i-C₃H₇ |
| 313 | CH₃ | 4-OC₆H₄-3-CF₃ | n-C₄H₉ |
| 314 | CH₃ | 4-OC₆H₄-3-CF₃ | n-C₆H₁₃ |
| 315 | CH₃ | 4-OC₆H₄-3-CF₃ | CH₂F |
| 316 | CH₃ | 4-OC₆H₄-3-CF₃ | CHF₂ |
| 317 | CH₃ | 4-OC₆H₄-3-CF₃ | CH₂CF₃ |
| 318 | CH₃ | 4-OC₆H₄-3-CF₃ | CH₂CH=CH₂ |
| 319 | CH₃ | 4-OC₆H₄-3-CF₃ | CH₂CH=CHCH₃ |
| 320 | CH₃ | 4-OC₆H₄-3-CF₃ | CH₂CH=C(CH₃)₂ |
| 321 | CH3 | 4-OC₆H₄-3-CF₃ | CH₂CH=CHCl |
| 322 | CH₃ | 4-OC₆H₄-3-CF₃ | CH₂CH=CCl₂ |
| 323 | CH₃ | 4-OC₆H₄-3-CF₃ | CH₂C(CH₃)=CH₂ |
| 324 | CH₃ | 4-OC₆H₄-3-CF₃ | CH₂C≡CH |
| 325 | CH₃ | 4-OC₆H₄-3-CF₃ | CH₂Si(CH₃)₃ |
| 326 | CH₃ | 4-OC₆H₄-3-CF₃ | CH₂-c.propyl-2,2-Cl₂ |
| 327 | CH₃ | 4-OC₆H₄-3-CF₃ | CH₂CN |
| 328 | CH₃ | 4-OC₆H₄-3-CF₃ | CH₂COOC₂H₅ |
| 329 | CH₃ | 4-OC₆H₄-3-CF₃ | CH(CH₃)COOC₂H₅ |
| 330 | CH₃ | 4-OC₆H₄-3-CF₃ | CH₂C₆H₄-3-CF₃ |
| 331 | CH₃ | 4-OC₆H₄-3-CF₃ | CH₂C₆H₄-4-F |
| 332 | CH₃ | 4-OC₆H₄-3-CF₃ | CH₂C₆H₄-3-F |
| 333 | CH₃ | 4-OC₆H₄-3-CF₃ | CH₂C₆H₄-2-F |
| 334 | CH₃ | 4-OC₆H₄-3-CF₃ | C(=O)OC₂H₅ |
| 335 | CH₃ | 4-OC₆H₄-3-CF₃ | C(=O)NHCH₃ |
| 336 | CH₃ | 4-OC₆H₄-3-CF₃ | C(=O)C(=O)OC₂H₅ |
| 337 | C₂H₅ | 4-OCH₂C₆H₄-3-CF₃ | CH₃ |
| 338 | C₂H₅ | 4-OCH₂C₆H₄-3-CF₃ | C₂H₅ |
| 339 | C₂H₅ | 4-OCH₂C₆H₄-3-CF₃ | n-C₃H₇ |
| 340 | C₂H₅ | 4-OCH₂C₆H₄-3-CF₃ | i-C₃H₇ |
| 341 | C₂H₅ | 4-OCH₂C₆H₄-3-CF₃ | n-C₄H₉ |
| 342 | C₂H₅ | 4-OCH₂C₆H₄-3-CF₃ | n-C₆H₁₃ |
| 343 | C₂H₅ | 4-OCH₂C₆H₄-3-CF₃ | CH₂F |
| 344 | C₂H₅ | 4-OCH₂C₆H₄-3-CF₃ | CHF₂ |
| 345 | C₂H₅ | 4-OCH₂C₆H₄-3-CF₃ | CH₂CF₃ |
| 346 | C₂H₅ | 4-OCH₂C₆H₄-3-CF₃ | CH₂CH=CH₂ |
| 347 | C₂H₅ | 4-OCH₂C₆H₄-3-CF₃ | CH₂CH=CHCH₃ |
| 348 | C₂H₅ | 4-OCH₂C₆H₄-3-CF₃ | CH₂CH=C(CH₃)₂ |
| 349 | C₂H₅ | 4-OCH₂C₆H₄-3-CF₃ | CH₂CH=CHCl |
| 350 | C₂H₅ | 4-OCH₂C₆H₄-3-CF₃ | CH₂CH=CCl₂ |
| 351 | C₂H₅ | 4-OCH₂C₆H₄-3-CF₃ | CH₂C(CH₃)=CH₂ |
| 352 | C₂H₅ | 4-OCH₂C₆H₄-3-CF₃ | CH₂C≡CH |
| 353 | C₂H₅ | 4-OCH₂C₆H₄-3-CF₃ | CH₂Si(CH₃)₃ |
| 354 | C₂H₅ | 4-OCH₂C₆H₄-3-CF₃ | CH₂-c.propyl-2,2-Cl₂ |
| 355 | C₂H₅ | 4-OCH₂C₆H₄-3-CF₃ | CH₂CN |
| 356 | C₂H₅ | 4-OCH₂C₆H₄-3-CF₃ | CH₂COOC₂H₅ |
| 357 | C₂H₅ | 4-OCH₂C₆H₄-3-CF₃ | CH(CH₃)COOC₂H₅ |
| 358 | C₂H₅ | 4-OCH₂C₆H₄-3-CF₃ | CH₂C₆H₄-3-CF₃ |
| 359 | C₂H₅ | 4-OCH₂C₆H₄-3-CF₃ | CH₂C₆H₄-4-F |
| 360 | C₂H₅ | 4-OCH₂C₆H₄-3-CF₃ | CH₂C₆H₄-3-F |
| 361 | C₂H₅ | 4-OCH₂C₆H₄-3-CF₃ | CH₂C₆H₄-2-F |
| 362 | C₂H₅ | 4-OC₆H₄-3-CF₃ | C(=O)OC₂H₅ |
| 363 | C₂H₅ | 4-OCH₂C₆H₄-3-CF₃ | C(=O)NHCH₃ |
| 364 | C₂H₅ | 4-OCH₂C₆H₄-3-CF₃ | C(=O)C(=O)OC₂H₅ |
| 365 | CH₃ | 4-OC₆H₄-4-Cl | CH₃ |
| 366 | CH₃ | 4-OC₆H₄-4-Cl | C₂H₅ |
| 367 | CH₃ | 4-OC₆H₄-4-Cl | n-C₃H₇ |
| 368 | CH₃ | 4-OC₆H₄-4-Cl | i-C₃H₇ |
| 369 | CH₃ | 4-OC₆H₄-4-Cl | n-C₄H₉ |
| 370 | CH₃ | 4-OC₆H₄-4-Cl | n-C₆H₁₃ |
| 371 | CH₃ | 4-OC₆H₄-4-Cl | CH₂F |
| 372 | CH₃ | 4-OC₆H₄-4-Cl | CHF₂ |
| 373 | CH₃ | 4-OC₆H₄-4-Cl | CH₂CF₃ |
| 374 | CH₃ | 4-OC₆H₄-4-Cl | CH₂CH=CH₂ |
| 375 | CH₃ | 4-OC₆H₄-4-Cl | CH₂CH=CHCH₃ |
| 376 | CH₃ | 4-OC₆H₄-4-Cl | CH₂CH=C(CH₃)₂ |
| 377 | CH₃ | 4-OC₆H₄-4-Cl | CH₂CH=CHCl |
| 378 | CH₃ | 4-OC₆H₄-4-Cl | CH₂CH=CCl₂ |
| 379 | CH₃ | 4-OC₆H₄-4-Cl | CH₂C(CH₃)=CH₂ |
| 380 | CH₃ | 4-OC₆H₄-4-Cl | CH₂C≡CH |
| 381 | CH₃ | 4-OC₆H₄-4-Cl | CH₂Si(CH₃)₃ |
| 382 | CH₃ | 4-OC₆H₄-4-Cl | CH₂-c.propyl-2,2-Cl₂ |
| 383 | CH₃ | 4-OC₆H₄-4-Cl | CH₂CN |
| 384 | CH₃ | 4-OC₆H₄-4-Cl | CH₂COOC₂H₅ |
| 385 | CH₃ | 4-OC₆H₄-4-Cl | CH(CH₃)COOC₂H₅ |
| 386 | CH₃ | 4-OC₆H₄-4-Cl | CH₂C₆H₄-3-CF₃ |
| 387 | CH₃ | 4-OC₆H₄-4-Cl | CH₂C₆H₄-4-F |
| 388 | CH₃ | 4-OC₆H₄-4-Cl | CH₂C₆H₄-3-F |
| 389 | CH₃ | 4-OC₆H₄-4-Cl | CH₂C₆H₄-2-F |
| 390 | CH₃ | 4-OC₆H₄-4-Cl | C(=O)OC₂H₅ |
| 391 | CH₃ | 4-OC₆H₄-4-Cl | C(=O)NHCH₃ |
| 392 | CH₃ | 4-OC₆H₄-4-Cl | C(=O)C(=O)OC₂H₅ |
| 393 | CH₃ | 4-OC₆H₄-3-Cl | CH₃ |
| 394 | CH₃ | 4-OC₆H₄-3-Cl | C₂H₅ |
| 395 | CH₃ | 4-OC₆H₄-3-Cl | n-C₃H₇ |
| 396 | CH₃ | 4-OC₆H₄-3-Cl | i-C₃H₇ |
| 397 | CH₃ | 4-OC₆H₄-3-Cl | n-C₄H₉ |
| 398 | CH₃ | 4-OC₆H₄-3-Cl | n-C₆H₁₃ |
| 399 | CH₃ | 4-OC₆H₄-3-Cl | CH₂F |
| 400 | CH₃ | 4-OC₆H₄-3-Cl | CHF₂ |
| 401 | CH₃ | 4-OC₆H₄-3-Cl | CH₂CF₃ |
| 402 | CH₃ | 4-OC₆H₄-3-Cl | CH₂CH=CH₂ |
| 403 | CH₃ | 4-OC₆H₄-3-Cl | CH₂CH=CHCH₃ |
| 404 | CH₃ | 4-OC₆H₄-3-Cl | CH₂CH=C(CH₃)₂ |
| 405 | CH₃ | 4-OC₆H₄-3-Cl | CH₂CH=CHCl |
| 406 | CH₃ | 4-OC₆H₄-3-Cl | CH₂CH=CCl₂ |
| 407 | CH₃ | 4-OC₆H₄-3-Cl | CH₂C(CH₃)=CH₂ |
| 408 | CH₃ | 4-OC₆H₄-3-Cl | CH₂C≡CH |
| 409 | CH₃ | 4-OC₆H₄-3-Cl | CH₂Si(CH₃)₃ |
| 410 | CH₃ | 4-OC₆H₄-3-Cl | CH₂-c.propyl-2,2-Cl₂ |
| 411 | CH₃ | 4-OC₆H₄-3-Cl | CH₂CN |
| 412 | CH₃ | 4-OC₆H₄-3-Cl | CH₂COOC₂H₅ |
| 413 | CH₃ | 4-OC₆H₄-3-Cl | CH(CH₃)COOC₂H₅ |
| 414 | CH₃ | 4-OC₆H₄-3-Cl | CH₂C₆H₄-3-CF₃ |
| 415 | CH₃ | 4-OC₆H₄-3-Cl | CH₂C₆H₄-4-F |
| 416 | CH₃ | 4-OC₆H₄-3-Cl | CH₂C₆H₄-3-F |
| 417 | CH₃ | 4-OC₆H₄-3-Cl | CH₂C₆H₄-2-F |
| 418 | CH₃ | 4-OC₆H₄-3-Cl | C(=O)OC₂H₅ |
| 419 | CH₃ | 4-OC₆H₄-3-Cl | C(=O)NHCH₃ |
| 420 | CH₃ | 4-OC₆H₄-3-Cl | C(=O)C(=O)OC₂H₅ |
| 421 | CH₃ | 4-OC₆H₄-2-Cl | CH₃ |
| 242 | CH₃ | 4-OC₆H₄-2-Cl | C₂H₅ |
| 423 | CH₃ | 4-OC₆H₄-2-Cl | n-C₃H₇ |
| 424 | CH₃ | 4-OC₆H₄-2-Cl | i-C₃H₇ |
| 425 | CH₃ | 4-OC₆H₄-2-Cl | n-C₄H₉ |
| 426 | CH₃ | 4-OC₆H₄-2-Cl | n-C₆H₁₃ |
| 427 | CH₃ | 4-OC₆H₄-2-Cl | CH₂F |
| 428 | CH₃ | 4-OC₆H₄-2-Cl | CHF₂ |
| 429 | CH₃ | 4-OC₆H₄-2-Cl | CH₂CF₃ |
| 430 | CH₃ | 4-OC₆H₄-2-Cl | CH₂CH=CH₂ |
| 431 | CH₃ | 4-OC₆H₄-2-Cl | CH₂CH=CHCH₃ |
| 432 | CH₃ | 4-OC₆H₄-2-Cl | CH₂CH=C(CH₃)₂ |
| 433 | CH₃ | 4-OC₆H₄-2-Cl | CH₂CH=CHCl |
| 434 | CH₃ | 4-OC₆H₄-2-Cl | CH₂CH=CCl₂ |
| 435 | CH₃ | 4-OC₆H₄-2-Cl | CH₂C(CH₃)=CH₂ |
| 436 | CH₃ | 4-OC₆H₄-2-Cl | CH₂C≡CH |
| 437 | CH₃ | 4-OC₆H₄-2-Cl | CH₂Si(CH₃)₃ |
| 438 | CH₃ | 4-OC₆H₄-2-Cl | CH₂-c.propyl-2,2-Cl₂ |
| 439 | CH₃ | 4-OC₆H₄-2-Cl | CH₂CN |
| 440 | CH₃ | 4-OC₆H₄-2-Cl | CH₂COOC₂H₅ |
| 441 | CH₃ | 4-OC₆H₄-2-Cl | CH(CH₃)COOC₂H₅ |
| 442 | CH₃ | 4-OC₆H₄-2-Cl | CH₂C₆H₄-3-CF₃ |
| 443 | CH₃ | 4-OC₆H₄-2-Cl | CH₂C₆H₄-4-F |
| 444 | CH₃ | 4-OC₆H₄-2-Cl | CH₂C₆H₄-3-F |
| 445 | CH₃ | 4-OC₆H₄-2-Cl | CH₂C₆H₄-2-F |
| 446 | CH₃ | 4-OC₆H₄-2-Cl | C(=O)OC₂H₅ |
| 447 | CH₃ | 4-OC₆H₄-2-Cl | C(=O)NHCH₃ |
| 448 | CH₃ | 4-OC₆H₄-2-Cl | C(=O)C(=O)OC₂H₅ |
| 449 | CH₃ | 4-OC₆H₄-4-F | CH₃ |
| 450 | CH₃ | 4-OC₆H₄-4-F | C₂H₅ |
| 451 | CH₃ | 4-OC₆H₄-4-F | n-C₃H₇ |
| 452 | CH₃ | 4-OC₆H₄-4-F | i-C₃H₇ |
| 453 | CH₃ | 4-OC₆H₄-4-F | n-C₄H₉ |
| 454 | CH₃ | 4-OC₆H₄-4-F | n-C₆H₁₃ |
| 455 | CH₃ | 4-OC₆H₄-4-F | CH₂F |
| 456 | CH₃ | 4-OC₆H₄-4-F | CHF₂ |
| 457 | CH₃ | 4-OC₆H₄-4-F | CH₂CF₃ |
| 458 | CH₃ | 4-OC₆H₄-4-F | CH₂CH=CH₂ |
| 459 | CH₃ | 4-OC₆H₄-4-F | CH₂CH=CHCH₃ |
| 460 | CH₃ | 4-OC₆H₄-4-F | CH₂CH=C(CH₃)₂ |
| 461 | CH₃ | 4-OC₆H₄-4-F | CH₂CH=CHCl |
| 462 | CH₃ | 4-OC₆H₄-4-F | CH₂CH=CCl₂ |
| 463 | CH₃ | 4-OC₆H₄-4-F | CH₂C(CH₃)=CH₂ |
| 464 | CH₃ | 4-OC₆H₄-4-F | CH₂C≡CH |
| 465 | CH₃ | 4-OC₆H₄-4-F | CH₂Si(CH₃)₃ |
| 466 | CH₃ | 4-OC₆H₄-4-F | CH₂-c.propyl-2,2-Cl₂ |
| 467 | CH₃ | 4-OC₆H₄-4-F | CH₂CN |
| 468 | CH₃ | 4-OC₆H₄-4-F | CH₂COOC₂H₅ |
| 469 | CH₃ | 4-OC₆H₄-4-F | CH(CH₃)COOC₂H₅ |
| 470 | CH₃ | 4-OC₆H₄-4-F | CH₂C₆H₄-3-CF₃ |
| 471 | CH₃ | 4-OC₆H₄-4-F | CH₂C₆H₄-4-F |
| 472 | CH₃ | 4-OC₆H₄-4-F | CH₂C₆H₄-3-F |
| 473 | CH₃ | 4-OC₆H₄-4-F | CH₂C₆H₄-2-F |
| 474 | CH₃ | 4-OC₆H₄-4-F | C(=O)OC₂H₅ |
| 475 | CH₃ | 4-OC₆H₄-4-F | C(=O)NHCH₃ |
| 476 | CH₃ | 4-OC₆H₄-4-F | C(=O)C(=O)OC₂H₅ |
| 477 | CH₃ | 4-OC₆H₄-3-F | CH₃ |
| 478 | CH₃ | 4-OC₆H₄-3-F | C₂H₅ |
| 479 | CH₃ | 4-OC₆H₄-3-F | n-C₃H₇ |
| 480 | CH₃ | 4-OC₆H₄-3-F | i-C₃H₇ |
| 481 | CH₃ | 4-OC₆H₄-3-F | n-C₄H₉ |
| 482 | CH₃ | 4-OC₆H₄-3-F | n-C₆H₁₃ |
| 483 | CH₃ | 4-OC₆H₄-3-F | CH₂F |
| 484 | CH₃ | 4-OC₆H₄-3-F | CHF₂ |
| 485 | CH₃ | 4-OC₆H₄-3-F | CH₂CF₃ |
| 486 | CH₃ | 4-OC₆H₄-3-F | CH₂CH=CH₂ |
| 487 | CH₃ | 4-OC₆H₄-3-F | CH₂CH=CHCH₃ |
| 488 | CH₃ | 4-OC₆H₄-3-F | CH₂CH=C(CH₃)₂ |
| 489 | CH₃ | 4-OC₆H₄-3-F | CH₂CH=CHCl |
| 490 | CH₃ | 4-OC₆H₄-3-F | CH₂CH=CCl₂ |
| 491 | CH₃ | 4-OC₆H₄-3-F | CH₂C(CH₃)=CH₂ |
| 492 | CH₃ | 4-OC₆H₄-3-F | CH₂C≡CH |
| 493 | CH₃ | 4-OC₆H₄-3-F | CH₂Si(CH₃)₃ |
| 494 | CH₃ | 4-OC₆H₄-3-F | CH₂-c.propyl-2,2-Cl₂ |
| 495 | CH₃ | 4-OC₆H₄-3-F | CH₂CN |
| 496 | CH₃ | 4-OC₆H₄-3-F | CH₂COOC₂H₅ |
| 497 | CH₃ | 4-OC₆H₄-3-F | CH(CH₃)COOC₂H₅ |
| 498 | CH₃ | 4-OC₆H₄-3-F | CH₂C₆H₄-3-CF₃ |
| 499 | CH₃ | 4-OC₆H₄-3-F | CH₂C₆H₄-4-F |
| 500 | CH₃ | 4-OC₆H₄-3-F | CH₂C₆H₄-3-F |
| 501 | CH₃ | 4-OC₆H₄-3-F | CH₂C₆H₄-2-F |
| 502 | CH₃ | 4-OC₆H₄-3-F | C(=O)OC₂H₅ |
| 503 | CH₃ | 4-OC₆H₄-3-F | C(=O)NHCH₃ |
| 504 | CH₃ | 4-OC₆H₄-3-F | C(=O)C(=O)OC₂H₅ |
| 505 | CH₃ | 4-OC₆H₄-2-F | CH₃ |
| 506 | CH₃ | 4-OC₆H₄-2-F | C₂H₅ |
| 507 | CH₃ | 4-OC₆H₄-2-F | n-C₃H₇ |
| 508 | CH₃ | 4-OC₆H₄-2-F | i-C₃H₇ |
| 509 | CH₃ | 4-OC₆H₄-2-F | n-C₄H₉ |
| 510 | CH₃ | 4-OC₆H₄-2-F | n-C₆H₁₃ |
| 511 | CH₃ | 4-OC₆H₄-2-F | CH₂F |
| 512 | CH₃ | 4-OC₆H₄-2-F | CHF₂ |
| 513 | CH₃ | 4-OC₆H₄-2-F | CH₂CF₃ |
| 514 | CH₃ | 4-OC₆H₄-2-F | CH₂CH=CH₂ |
| 515 | CH₃ | 4-OC₆H₄-2-F | CH₂CH=CHCH₃ |
| 516 | CH₃ | 4-OC₆H₄-2-F | CH₂CH=C(CH₃)₂ |
| 517 | CH₃ | 4-OC₆H₄-2-F | CH₂CH=CHCl |
| 518 | CH₃ | 4-OC₆H₄-2-F | CH₂CH=CCl₂ |
| 519 | CH₃ | 4-OC₆H₄-2-F | CH₂C(CH₃)=CH₂ |
| 520 | CH₃ | 4-OC₆H₄-2-F | CH₂C≡CH |
| 521 | CH₃ | 4-OC₆H₄-2-F | CH₂Si(CH₃)₃ |
| 522 | CH₃ | 4-OC₆H₄-2-F | CH₂-c,propyl-2,2-Cl₂ |
| 523 | CH₃ | 4-OC₆H₄-2-F | CH₂CN |
| 524 | CH₃ | 4-OC₆H₄-2-F | CH₂COOC₂H₅ |
| 525 | CH₃ | 4-OC₆H₄-2-F | CH(CH₃)COOC₂H₅ |
| 526 | CH₃ | 4-OC₆H₄-2-F | CH₂C₆H₄-3-CF₃ |
| 527 | CH₃ | 4-OC₆H₄-2-F | CH₂C₆H₄-4-F |
| 528 | CH₃ | 4-OC₆H₄-2-F | CH₂C₆H₄-3-F |
| 529 | CH₃ | 4-OC₆H₄-2-F | CH₂C₆H₄-2-F |
| 530 | CH₃ | 4-OC₆H₄-2-F | C(=O)OC₂H₅ |
| 531 | CH₃ | 4-OC₆H₄-2-F | C(=O)NHCH₃ |
| 532 | CH₃ | 4-OC₆H₄-2-F | C(=O)C(=O)OC₂H₅ |
| 533 | CH₃ | 4-OC₆H₄-4-Br | CH₃ |
| 534 | CH₃ | 4-OC₆H₄-4-Br | C₂H₅ |
| 535 | CH₃ | 4-OC₆H₄-4-Br | n-C₃H₇ |
| 536 | CH₃ | 4-OC₆H₄-4-Br | i-C₃H₇ |
| 537 | CH₃ | 4-OC₆H₄-4-Br | n-C₄H₉ |
| 538 | CH₃ | 4-OC₆H₄-4-Br | n-C₆H₁₃ |
| 539 | CH₃ | 4-OC₆H₄-4-Br | CH₂F |
| 540 | CH₃ | 4-OC₆H₄-4-Br | CHF₂ |
| 541 | CH₃ | 4-OC₆H₄-4-Br | CH₂CF₃ |
| 542 | CH₃ | 4-OC₆H₄-4-Br | CH₂CH=CH₂ |
| 543 | CH₃ | 4-OC₆H₄-4-Br | CH₂CH=CHCH₃ |
| 544 | CH₃ | 4-OC₆H₄-4-Br | CH₂CH=C(CH₃)₂ |
| 545 | CH₃ | 4-OC₆H₄-4-Br | CH₂CH=CHCl |
| 546 | CH₃ | 4-OC₆H₄-4-Br | CH₂CH=CCl₂ |
| 547 | CH₃ | 4-OC₆H₄-4-Br | CH₂C(CH₃)=CH₂ |
| 548 | CH₃ | 4-OC₆H₄-4-Br | CH₂C≡CH |
| 549 | CH₃ | 4-OC₆H₄-4-Br | CH₂Si(CH₃)₃ |
| 550 | CH₃ | 4-OC₆H₄-4-Br | CH₂-c.propyl-2,2-Cl₂ |
| 551 | CH₃ | 4-OC₆H₄-4-Br | CH₂CN |
| 552 | CH₃ | 4-OC₆H₄-4-Br | CH₂COOC₂H₅ |
| 553 | CH₃ | 4-OC₆H₄-4-Br | CH(CH₃)COOC₂H₅ |
| 554 | CH₃ | 4-OC₆H₄-4-Br | CH₂C₆H₄-3-CF₃ |
| 555 | CH₃ | 4-OC₆H₄-4-Br | CH₂C₆H₄-4-F |
| 556 | CH₃ | 4-OC₆H₄-4-Br | CH₂C₆H₄-3-F |
| 557 | CH₃ | 4-OC₆H₄-4-Br | CH₂C₆H₄-2-F |
| 558 | CH₃ | 4-OC₆H₄-4-Br | C(=O)OC₂H₅ |
| 559 | CH₃ | 4-OC₆H₄-4-Br | C(=O)NHCH₃ |
| 560 | CH₃ | 4-OC₆H₄-4-Br | C(=O)C(=O)OC₂H₅ |
| 561 | CH₃ | 4-OC₆H₄-3-Br | CH₃ |
| 562 | CH₃ | 4-OC₆H₄-3-Br | C₂H₅ |
| 563 | CH₃ | 4-OC₆H₄-3-Br | n-C₃H₇ |
| 564 | CH₃ | 4-OC₆H₄-3-Br | i-C₃H₇ |
| 565 | CH₃ | 4-OC₆H₄-3-Br | n-C₄H₉ |
| 566 | CH₃ | 4-OC₆H₄-3-Br | n-C₆H₁₃ |
| 567 | CH₃ | 4-OC₆H₄-3-Br | CH₂F |
| 568 | CH₃ | 4-OC₆H₄-3-Br | CHF₂ |
| 569 | CH₃ | 4-OC₆H₄-3-Br | CH₂CF₃ |
| 570 | CH₃ | 4-OC₆H₄-3-Br | CH₂CH=CH₂ |
| 571 | CH₃ | 4-OC₆H₄-3-Br | CH₂CH=CHCH₃ |
| 572 | CH₃ | 4-OC₆H₄-3-Br | CH₂CH=C(CH₃)₂ |
| 573 | CH₃ | 4-OC₆H₄-3-Br | CH₂CH=CHCl |
| 574 | CH₃ | 4-OC₆H₄-3-Br | CH₂CH=CCl₂ |
| 575 | CH₃ | 4-OC₆H₄-3-Br | CH₂C(CH₃)=CH₂ |
| 576 | CH₃ | 4-OC₆H₄-3-Br | CH₂C≡CH |
| 577 | CH₃ | 4-OC₆H₄-3-Br | CH₂Si(CH₃)₃ |
| 578 | CH₃ | 4-OC₆H₄-3-Br | CH₂-c.propyl-2,2-Cl₂ |
| 579 | CH₃ | 4-OC₆H₄-3-Br | CH₂CN |
| 580 | CH₃ | 4-OC₆H₄-3-Br | CH₂COOC₂H₅ |
| 581 | CH₃ | 4-OC₆H₄-3-Br | CH(CH₃)COOC₂H₅ |
| 582 | CH₃ | 4-OC₆H₄-3-Br | CH₂C₆H₄-3-CF₃ |
| 583 | CH₃ | 4-OC₆H₄-3-Br | CH₂C₆H₄-4-F |
| 584 | CH₃ | 4-OC₆H₄-3-Br | CH₂C₆H₄-3-F |
| 585 | CH₃ | 4-OC₆H₄-3-Br | CH₂C₆H₄-2-F |
| 586 | CH₃ | 4-OC₆H₄-3-Br | C(=O)OC₂H₅ |
| 587 | CH₃ | 4-OC₆H₄-3-Br | C(=O)NHCH₃ |
| 588 | CH₃ | 4-OC₆H₄-3-Br | C(=O)C(=O)OC₂H₅ |
| 589 | CH₃ | 4-OC₆H₄-2-Br | CH₃ |
| 590 | CH₃ | 4-OC₆H₄-2-Br | C₂H₅ |
| 591 | CH₃ | 4-OC₆H₄-2-Br | n-C₃H₇ |
| 592 | CH₃ | 4-OC₆H₄-2-Br | i-C₃H₇ |
| 593 | CH₃ | 4-OC₆H₄-2-Br | n-C₄H₉ |
| 594 | CH₃ | 4-OC₆H₄-2-Br | n-C₆H₁₃ |
| 595 | CH₃ | 4-OC₆H₄-2-Br | CH₂F |
| 596 | CH₃ | 4-OC₆H₄-2-Br | CHF₂ |
| 597 | CH₃ | 4-OC₆H₄-2-Br | CH₂CF₃ |
| 598 | CH₃ | 4-OC₆H₄-2-Br | CH₂CH=CH₂ |
| 599 | CH₃ | 4-OC₆H₄-2-Br | CH₂CH=CHCH₃ |
| 600 | CH₃ | 4-OC₆H₄-2-Br | CH₂CH=C(CH₃)₂ |
| 601 | CH₃ | 4-OC₆H₄-2-Br | CH₂CH=CHCl |
| 602 | CH₃ | 4-OC₆H₄-2-Br | CH₂CH=CCl₂ |
| 603 | CH₃ | 4-OC₆H₄-2-Br | CH₂C(CH₃)=CH₂ |
| 604 | CH₃ | 4-OC₆H₄-2-Br | CH₂C≡CH |
| 605 | CH₃ | 4-OC₆H₄-2-Br | CH₂Si(CH₃)₃ |
| 606 | CH₃ | 4-OC₆H₄-2-Br | CH₂-c.propyl-2,2-Cl₂ |
| 607 | CH₃ | 4-OC₆H₄-2-Br | CH₂CN |
| 608 | CH₃ | 4-OC₆H₄-2-Br | CH₂COOC₂H₅ |
| 609 | CH₃ | 4-OC₆H₄-2-Br | CH(CH₃)COOC₂H₅ |
| 610 | CH₃ | 4-OC₆H₄-2-Br | CH₂C₆H₄-3-CF₃ |
| 611 | CH₃ | 4-OC₆H₄-2-Br | CH₂C₆H₄-4-F |
| 612 | CH₃ | 4-OC₆H₄-2-Br | CH₂C₆H₄-3-F |
| 613 | CH₃ | 4-OC₆H₄-2-Br | CH₂C₆H-2-F |
| 614 | CH₃ | 4-OC₆H₄-2-Br | C(=O)OC₂H₅ |
| 615 | CH₃ | 4-OC₆H₄-2-Br | C(=O)NHCH₃ |
| 616 | CH₃ | 4-OC₆H₄-2-Br | C(=O)C(=O)OC₂H₅ |
| 617 | CH₃ | 4-OC₆H₃-2,4-Cl₂ | CH₃ |
| 618 | CH₃ | 4-OC₆H₃-2,4-Cl₂ | C₂H₅ |
| 619 | CH₃ | 4-OC₆H₃-2,4-Cl₂ | n-C₃H₇ |
| 620 | CH₃ | 4-OC₆H₃-2,4-Cl₂ | i-C₃H₇ |
| 621 | CH₃ | 4-OC₆H₃-2,4-Cl₂ | n-C₄H₉ |
| 622 | CH₃ | 4-OC₆H₃-2,4-Cl₂ | n-C₆H₁₃ |
| 623 | CH₃ | 4-OC₆H₃-2,4-Cl₂ | CH₂F |
| 624 | CH₃ | 4-OC₆H₃-2,4-Cl₂ | CHF₂ |
| 625 | CH₃ | 4-OC₆H₃-2,4-Cl₂ | CH₂CF₃ |
| 626 | CH₃ | 4-OC₆H₃-2,4-Cl₂ | CH₂CH=CH₂ |
| 627 | CH₃ | 4-OC₆H₃-2,4-Cl₂ | CH₂CH=CHCH₃ |
| 628 | CH₃ | 4-OC₆H₃-2,4-Cl₂ | CH₂CH=C(CH₃)₂ |
| 629 | CH₃ | 4-OC₆H₃-2,4-Cl₂ | CH₂CH=CHCl |
| 630 | CH₃ | 4-OC₆H₃-2,4-Cl₂ | CH₂CH=CCl₂ |
| 631 | CH₃ | 4-OC₆H₃-2,4-Cl₂ | CH₂C(CH₃)=CH₂ |
| 632 | CH₃ | 4-OC₆H₃-2,4-Cl₂ | CH₂C≡CH |
| 633 | CH₃ | 4-OC₆H₃-2,4-Cl₂ | CH₂Si(CH₃)₃ |
| 634 | CH₃ | 4-OC₆H₃-2,4-Cl₂ | CH₂-c.propyl-2,2-Cl₂ |
| 635 | CH₃ | 4-OC₆H₃-2,4-Cl₂ | CH₂CN |
| 636 | CH₃ | 4-OC₆H₃-2,4-Cl₂ | CH₂COOC₂H₅ |
| 637 | CH₃ | 4-OC₆H₃-2,4-Cl₂ | CH(CH₃)COOC₂H₅ |
| 638 | CH₃ | 4-OC₆H₃-2,4-Cl₂ | CH₂C₆H₄-3-CF₃ |
| 639 | CH₃ | 4-OC₆H₃-2,4-Cl₂ | CH₂C₆H₄-4-F |
| 640 | CH₃ | 4-OC₆H₃-2,4-Cl₂ | CH₂C₆H₄-3-F |
| 641 | CH₃ | 4-OC₆H₃-2,4-Cl₂ | CH₂C₆H₄-2-F |
| 642 | CH₃ | 4-OC₆H₃-2,4-Cl₂ | C(=O)OC₂H₅ |
| 643 | CH₃ | 4-OC₆H₃-2,4-Cl₂ | C(=O)NHCH₃ |
| 644 | CH₃ | 4-OC₆H₃-2,4-Cl₂ | C(=O)C(=O)OC₂H₅ |
| 645 | CH₃ | 4-OC₆H₃-3,4-Cl₂ | CH₃ |
| 646 | CH₃ | 4-OC₆H₃-3,4-Cl₂ | C₂H₅ |
| 647 | CH₃ | 4-OC₆H₃-3,4-Cl₂ | n-C₃H₇ |
| 648 | CH₃ | 4-OC₆H₃-3,4-Cl₂ | i-C₃H₇ |
| 649 | CH₃ | 4-OC₆H₃-3,4-Cl₂ | n-C₄H₉ |
| 650 | CH₃ | 4-OC₆H₃-3,4-Cl₂ | n-C₆H₁₃ |
| 651 | CH₃ | 4-OC₆H₃-3,4-Cl₂ | CH₂F |
| 652 | CH₃ | 4-OC₆H₃-3,4-Cl₂ | CHF₂ |
| 653 | CH₃ | 4-OC₆H₃-3,4-Cl₂ | CH₂CF₃ |
| 654 | CH₃ | 4-OC₆H₃-3,4-Cl₂ | CH₂CH=CH₂ |
| 655 | CH₃ | 4-OC₆H₃-3,4-Cl₂ | CH₂CH=CHCH₃ |
| 656 | CH₃ | 4-OC₆H₃-3,4-Cl₂ | CH₂CH=C(CH₃)₂ |
| 657 | CH₃ | 4-OC₆H₃-3,4-Cl₂ | CH₂CH=CHCl |
| 658 | CH₃ | 4-OC₆H₃-3,4-Cl₂ | CH₂CH=CCl₂ |
| 659 | CH₃ | 4-OC₆H₃-3,4-Cl₂ | CH₂C(CH₃)=CH₂ |
| 660 | CH₃ | 4-OC₆H₃-3,4-Cl₂ | CH₂C≡CH |
| 661 | CH₃ | 4-OC₆H₃-3,4-Cl₂ | CH₂Si(CH₃)₃ |
| 662 | CH₃ | 4-OC₆H₃-3,4-Cl₂ | CH₂-c.propyl-2,2-Cl₂ |
| 663 | CH₃ | 4-OC₆H₃-3,4-Cl₂ | CH₂CN |
| 664 | CH₃ | 4-OC₆H₃-3,4-Cl₂ | CH₂COOC₂H₅ |
| 665 | CH₃ | 4-OC₆H₃-3,4-Cl₂ | CH(CH₃)COOC₂H₅ |
| 666 | CH₃ | 4-OC₆H₃-3,4-Cl₂ | CH₂C₆H₄-3-CF₃ |
| 667 | CH₃ | 4-OC₆H₃-3,4-Cl₂ | CH₂C₆H₄-4-F |
| 668 | CH₃ | 4-OC₆H₃-3,4-Cl₂ | CH₂C₆H₄-3-F |
| 669 | CH₃ | 4-OC₆H₃-3,4-Cl₂ | CH₂C₆H₄-2-F |
| 670 | CH₃ | 4-OC₆H₃-3,4-Cl₂ | C(=O)OC₂H₅ |
| 671 | CH₃ | 4-OC₆H₃-3,4-Cl₂ | C(=O)NHCH₃ |
| 672 | CH₃ | 4-OC₆H₃-3,4-Cl₂ | C(=O)C(=O)OC₂H₅ |
| 673 | CH₃ | 4-OC₆H₃-2-Cl,4-Br | CH₃ |
| 674 | CH₃ | 4-OC₆H₃-2-Cl.4-Br | C₂H₅ |
| 675 | CH₃ | 4-OC₆H₃-2-Cl,4-Br | n-C₃H₇ |
| 676 | CH₃ | 4-OC₆H₃-2-Cl,4-Br | i-C₃H₇ |
| 677 | CH₃ | 4-OC₆H₃-2-Cl,4-Br | n-C₄H₉ |
| 678 | CH₃ | 4-OC₆H₃-2-Cl,4-Br | n-C₆H₁₃ |
| 679 | CH₃ | 4-OC₆H₃-2-Cl,4-Br | CH₂F |
| 680 | CH₃ | 4-OC₆H₃-2-Cl,4-Br | CHF₂ |
| 681 | CH₃ | 4-OC₆H₃-2-Cl,4-Br | CH₂CF₃ |
| 682 | CH₃ | 4-OC₆H₃-2-Cl,4-Br | CH₂CH=CH₂ |
| 683 | CH₃ | 4-OC₆H₃-2-Cl,4-Br | CH₂CH=CHCH₃ |
| 684 | CH₃ | 4-OC₆H₃-2-Cl,4-Br | CH₂CH=C(CH₃)₂ |
| 685 | CH₃ | 4-OC₆H₃-2-Cl,4-Br | CH₂CH=CHCl |
| 686 | CH₃ | 4-OC₆H₃-2-Cl,4-Br | CH₂CH=CCl₂ |
| 687 | CH₃ | 4-OC₆H₃-2-Cl,4-Br | CH₂C(CH₃)=CH₂ |
| 688 | CH₃ | 4-OC₆H₃-2-Cl,4-Br | CH₂C≡CH |
| 689 | CH₃ | 4-OC₆H₃-2-Cl,4-Br | CH₂Si(CH₃)₃ |
| 690 | CH₃ | 4-OC₆H₃-2-Cl,4-Br | CH₂-c.propyl-2,2-Cl₂ |
| 691 | CH₃ | 4-OC₆H₃-2-Cl,4-Br | CH₂CN |
| 692 | CH₃ | 4-OC₆H₃-2-Cl,4-Br | CH₂COOC₂H₅ |
| 693 | CH₃ | 4-OC₆H₃-2-Cl,4-Br | CH(CH₃)COOC₂H₅ |
| 694 | CH₃ | 4-OC₆H₃-2-Cl, 4-Br | CH₂C₆H₄-3-CF₃ |
| 695 | CH₃ | 4-OC₆H₃-2-Cl,4-Br | CH₂C₆H₄-4-F |
| 696 | CH₃ | 4-OC₆H₃-2-Cl,4-Br | CH₂C₆H₄-3-F |
| 697 | CH₃ | 4-OC₆H₃-2-Cl,4-Br | CH₂C₆H₄-2-F |
| 698 | CH₃ | 4-OC₆H₃-2-Cl,4-Br | C(=O)OC₂H₅ |
| 699 | CH₃ | 4-OC₆H₃-2-Cl,4-Br | C(=O)NHCH₃ |
| 700 | CH₃ | 4-OC₆H₃-2-Cl,4-Br | C(=O)C(=O)OC₂H₅ |
| 701 | CH₃ | 4-OC₆H₃-3,4-(-OCH₂O-) | CH₃ |
| 702 | CH₃ | 4-OC₆H₃-3,4-(-OCH₂O-) | C₂H₅ |
| 703 | CH₃ | 4-OC₆H₃-3,4-(-OCH₂O-) | n-C₃H₇ |
| 704 | CH₃ | 4-OC₆H₃-3,4-(-OCH₂O-) | i-C₃H₇ |
| 705 | CH₃ | 4-OC₆H₃-3,4-(-OCH₂O-) | n-C₄H₉ |
| 706 | CH₃ | 4-OC₆H₃-3,4-(-OCH₂O-) | n-C₆H₁₃ |
| 707 | CH₃ | 4-OC₆H₃-3,4-(-OCH₂O-) | CH₂F |
| 708 | CH₃ | 4-OC₆H₃-3,4-(-OCH₂O-) | CHF₂ |
| 709 | CH₃ | 4-OC₆H₃-3,4-(-OCH₂O-) | CH₂CF₃ |
| 710 | CH₃ | 4-OC₆H₃-3,4-(-OCH₂O-) | CH₂CH=CH₂ |
| 711 | CH₃ | 4-OC₆H₃-3,4-(-OCH₂O-) | CH₂CH=CHCH₃ |
| 712 | CH₃ | 4-OC₆H₃-3,4-(-OCH₂O-) | CH₂CH=C(CH₃)₂ |
| 713 | CH₃ | 4-OC₆H₃-3,4-(-OCH₂O-) | CH₂CH=CHCl |
| 714 | CH₃ | 4-OC₆H₃-3,4-(-OCH₂O-) | CH₂CH=CCl₂ |
| 715 | CH₃ | 4-OC₆H₃-3,4-(-OCH₂O-) | CH₂C(CH₃)=CH₂ |
| 716 | CH3 | 4-OC₆H₃-3,4-(-OCH₂O-) | CH₂C≡CH |
| 717 | CH₃ | 4-OC₆H₃-3,4-(-OCH₂O-) | CH₂Si(CH₃)₃ |
| 718 | CH₃ | 4-OC₆H₃-3,4-(-OCH₂O-) | CH₂-c.propyl-2,2-Cl₂ |
| 719 | CH₃ | 4-OC₆H₃-3,4-(-OCH₂O-) | CH₂CN |
| 720 | CH₃ | 4-OC₆H₃-3,4-(-OCH₂O-) | CH₂COOC₂H₅ |
| 721 | CH₃ | 4-OC₆H₃-3,4-(-OCH₂O-) | CH(CH₃)COOC₂H₅ |
| 722 | CH₃ | 4-OC₆H₃-3,4-(-OCH₂O-) | CH₂C₆H₄-3-CF₃ |
| 723 | CH₃ | 4-OC₆H₃-3,4-(-OCH₂O-) | CH₂C₆H₄-4-F |
| 724 | CH₃ | 4-OC₆H₃-3,4-(-OCH₂O-) | CH₂C₆H₄-3-F |
| 725 | CH₃ | 4-OC₆H₃-3,4-(-OCH₂O-) | CH₂C₆H₄-2-F |
| 726 | CH₃ | 4-OC₆H₃-3,4-(-OCH₂O-) | C(=O)OC₂H₅ |
| 727 | CH₃ | 4-OC₆H₃-3,4-(-OCH₂O-) | C(=O)NHCH₃ |
| 728 | CH₃ | 4-OC₆H₃-3,4-(-OCH₂O-) | C(=O)C(=O)OC₂H₅ |
| 729 | CH₃ | 4-OC₆H₄-4-SCH₃ | CH₃ |
| 730 | CH₃ | 4-OC₆H₄-4-SCH₃ | C₂H₅ |
| 731 | CH₃ | 4-OC₆H₄-4-SCH₃ | n-C₃H₇ |
| 732 | CH₃ | 4-OC₆H₄-4-SCH₃ | i-C₃H₇ |
| 733 | CH₃ | 4-OC₆H₄-4-SCH₃ | n-C₄H₉ |
| 734 | CH₃ | 4-OC₆H₄-4-SCH₃ | n-C₆H₁₃ |
| 735 | CH₃ | 4-OC₆H₄-4-SCH₃ | CH₂F |
| 736 | CH₃ | 4-OC₆H₄-4-SCH₃ | CHF₂ |
| 737 | CH₃ | 4-OC₆H₄-4-SCH₃ | CH₂CF₃ |
| 738 | CH₃ | 4-OC₆H₄-4-SCH₃ | CH₂CH=CH₂ |
| 739 | CH₃ | 4-OC₆H₄-4-SCH₃ | CH₂CH=CHCH₃ |
| 740 | CH₃ | 4-OC₆H₄-4-SCH₃ | CH₂CH=C(CH₃)₂ |
| 741 | CH₃ | 4-OC₆H₄-4-SCH₃ | CH₂CH=CHCl |
| 742 | CH₃ | 4-OC₆H₄-4-SCH₃ | CH₂CH=CCl₂ |
| 743 | CH₃ | 4-OC₆H₄-4-SCH₃ | CH₂C(CH₃)=CH₂ |
| 744 | CH₃ | 4-OC₆H₄-4-SCH₃ | CH₂C≡CH |
| 745 | CH₃ | 4-OC₆H₄-4-SCH₃ | CH₂Si(CH₃)₃ |
| 746 | CH₃ | 4-OC₆H₄-4-SCH₃ | CH₂-c.propyl-2,2-Cl₂ |
| 747 | CH₃ | 4-OC₆H₄-4-SCH₃ | CH₂CN |
| 748 | CH₃ | 4-OC₆H₄-4-SCH₃ | CH₂COOC₂H₅ |
| 749 | CH₃ | 4-OC₆H₄-4-SCH₃ | CH(CH₃)COOC₂H₅ |
| 750 | CH₃ | 4-OC₆H₄-4-SCH₃ | CH₂C₆H₄-3-CF₃ |
| 751 | CH₃ | 4-OC₆H₄-4-SCH₃ | CH₂C₆H₄-4-F |
| 752 | CH₃ | 4-OC₆H₄-4-SCH₃ | CH₂C₆H₄-3-F |
| 753 | CH₃ | 4-OC₆H₄-4-SCH₃ | CH₂C₆H₄-2-F |
| 754 | CH₃ | 4-OC₆H₄-4-SCH₃ | C(=O)OC₂H₅ |
| 755 | CH₃ | 4-OC₆H₄-4-SCH₃ | C(=O)NHCH₃ |
| 756 | CH₃ | 4-OC₆H₄-4-SCH₃ | C(=O)C(=O)OC₂H₅ |
| 757 | CH₃ | 4-OC₆H₄-4-OCH₃ | CH₃ |
| 758 | CH₃ | 4-OC₆H₄-4-OCH₃ | C₂H₅ |
| 759 | CH₃ | 4-OC₆H₄-4-OCH₃ | n-C₃H₇ |
| 760 | CH₃ | 4-OC₆H₄-4-OCH₃ | i-C₃H₇ |
| 761 | CH₃ | 4-OC₆H₄-4-OCH₃ | n-C₄H₉ |
| 762 | CH₃ | 4-OC₆H₄-4-OCH₃ | n-C₆H₁₃ |
| 763 | CH₃ | 4-OC₆H₄-4-OCH₃ | CH₂F |
| 764 | CH₃ | 4-OC₆H₄-4-OCH₃ | CHF₂ |
| 765 | CH₃ | 4-OC₆H₄-4-OCH₃ | CH₂CF₃ |
| 766 | CH₃ | 4-OC₆H₄-4-OCH₃ | CH₂CH=CH₂ |
| 767 | CH₃ | 4-OC₆H₄-4-OCH₃ | CH₂CH=CHCH₃ |
| 768 | CH₃ | 4-OC₆H₄-4-OCH₃ | CH₂CH=C(CH₃)₂ |
| 769 | CH₃ | 4-OC₆H₄-4-OCH₃ | CH₂CH=CHCl |
| 770 | CH₃ | 4-OC₆H₄-4-OCH₃ | CH₂CH=CCl₂ |
| 771 | CH₃ | 4-OC₆H₄-4-OCH₃ | CH₂C(CH₃)=CH₂ |
| 772 | CH₃ | 4-OC₆H₄-4-OCH₃ | CH₂C≡CH |
| 773 | CH₃ | 4-OC₆H₄-4-OCH₃ | CH₂Si(CH₃)₃ |
| 774 | CH₃ | 4-OC₆H₄-4-OCH₃ | CH₂-c.propyl-2,2-Cl₂ |
| 775 | CH₃ | 4-OC₆H₄-4-OCH₃ | CH₂CN |
| 776 | CH₃ | 4-OC₆H₄-4-OCH₃ | CH₂COOC₂H₅ |
| 777 | CH₃ | 4-OC₆H₄-4-OCH₃ | CH(CH₃)COOC₂H₅ |
| 778 | CH₃ | 4-OC₆H₄-4-OCH₃ | CH₂C₆H₄-3-CF₃ |
| 779 | CH₃ | 4-OC₆H₄-4-OCH₃ | CH₂C₆H₄-4-F |
| 780 | CH₃ | 4-OC₆H₄-4-OCH₃ | CH₂C₆H₄-3-F |
| 781 | CH₃ | 4-OC₆H₄-4-OCH₃ | CH₂C₆H₄-2-F |
| 782 | CH₃ | 4-OC₆H₄-4-OCH₃ | C(=O)OC₂H₅ |
| 783 | CH₃ | 4-OC₆H₄-4-OCH₃ | C(=O)NHCH₃ |
| 784 | CH₃ | 4-OC₆H₄-4-OCH₃ | C(=O)C(=O)OC₂H₅ |
| 785 | CH₃ | 4-OC₆H₄-4-t-butyl | CH₃ |
| 786 | CH₃ | 4-OC₆H₄-4-t-butyl | C₂H₅ |
| 787 | CH₃ | 4-OC₆H₄-4-t-butyl | n-C₃H₇ |
| 788 | CH₃ | 4-OC₆H₄-4-t-butyl | i-C₃H₇ |
| 789 | CH₃ | 4-OC₆H₄-4-t-butyl | n-C₄H₉ |
| 790 | CH₃ | 4-OC₆H₄-4-t-butyl | n-C₆H₁₃ |
| 791 | CH₃ | 4-OC₆H₄-4-t-butyl | CH₂F |
| 792 | CH₃ | 4-OC₆H₄-4-t-butyl | CHF₂ |
| 793 | CH₃ | 4-OC₆H₄-4-t-butyl | CH₂CF₃ |
| 794 | CH₃ | 4-OC₆H₄-4-t-butyl | CH₂CH=CH₂ |
| 795 | CH₃ | 4-OC₆H₄-4-t-butyl | CH₂CH=CHCH₃ |
| 796 | CH₃ | 4-OC₆H₄-4-t-butyl | CH₂CH=C(CH₃)₂ |
| 797 | CH₃ | 4-OC₆H₄-4-t-butyl | CH₂CH=CHCl |
| 798 | CH₃ | 4-OC₆H₄-4-t-butyl | CH₂CH=CCl₂ |
| 799 | CH₃ | 4-OC₆H₄-4-t-butyl | CH₂C(CH₃)=CH₂ |
| 800 | CH₃ | 4-OC₆H₄-4-t-butyl | CH₂C≡CH |
| 801 | CH₃ | 4-OC₆H₄-4-t-butyl | CH₂Si(CH₃)₃ |
| 802 | CH₃ | 4-OC₆H₄-4-t-butyl | CH₂-c.propyl-2,2-Cl₂ |
| 803 | CH₃ | 4-OC₆H₄-4-t-butyl | CH₂CN |
| 804 | CH₃ | 4-OC₆H₄-4-t-butyl | CH₂COOC₂H₅ |
| 805 | CH₃ | 4-OC₆H₄-4-t-butyl | CH(CH₃)COOC₂H₅ |
| 806 | CH₃ | 4-OC₆H₄-4-t-butyl | CH₂C₆H₄-3-CF₃ |
| 807 | CH₃ | 4-OC₆H₄-4-t-butyl | CH₂C₆H₄-4-F |
| 808 | CH₃ | 4-OC₆H₄-4-t-butyl | CH₂C₆H₄-3-F |
| 809 | CH₃ | 4-OC₆H₄-4-t-butyl | CH₂C₆H₄-2-F |
| 810 | CH₃ | 4-OC₆H₄-4-t-butyl | C(=O)OC₂H₅ |
| 811 | CH₃ | 4-OC₆H₄-4-t-butyl | C(=O)NHCH₃ |
| 812 | CH₃ | 4-OC₆H₄-4-t-butyl | C(=O)C(=O)OC₂H₅ |
| 813 | CH₃ | 4-OC₆H₄-4-CF₃ | CH₃ |
| 814 | CH₃ | 4-OC₆H₄-4-CF₃ | C₂H₅ |
| 815 | CH₃ | 4-OC₆H₄-4-CF₃ | n-C₃H₇ |
| 816 | CH₃ | 4-OC₆H₄-4-CF₃ | i-C₃H₇ |
| 817 | CH₃ | 4-OC₆H₄-4-CF₃ | n-C₄H₉ |
| 818 | CH₃ | 4-OC₆H₄-4-CF₃ | n-C₆H₁₃ |
| 819 | CH₃ | 4-OC₆H₄-4-CF₃ | CH₂F |
| 820 | CH₃ | 4-OC₆H₄-4-CF₃ | CHF₂ |
| 821 | CH₃ | 4-OC₆H₄-4-CF₃ | CH₂CF₃ |
| 822 | CH₃ | 4-OC₆H₄-4-CF₃ | CH₂CH=CH₂ |
| 823 | CH₃ | 4-OC₆H₄-4-CF₃ | CH₂CH=CHCH₃ |
| 824 | CH₃ | 4-OC₆H₄-4-CF₃ | CH₂CH=C(CH₃)₂ |
| 825 | CH3 | 4-OC₆H₄-4-CF₃ | CH₂CH=CHCl |
| 826 | CH₃ | 4-OC₆H₄-4-CF₃ | CH₂CH=CCl₂ |
| 827 | CH₃ | 4-OC₆H₄-4-CF₃ | CH₂C(CH₃)=CH₂ |
| 828 | CH₃ | 4-OC₆H₄-4-CF₃ | CH₂C≡CH |
| 829 | CH₃ | 4-OC₆H₄-4-CF₃ | CH₂Si(CH₃)₃ |
| 830 | CH₃ | 4-OC₆H₄-4-CF₃ | CH₂-c.propyl-2,2-Cl₂ |
| 831 | CH₃ | 4-OC₆H₄-4-CF₃ | CH₂CN |
| 832 | CH₃ | 4-OC₆H₄-4-CF₃ | CH₂COOC₂H₅ |
| 833 | CH₃ | 4-OC₆H₄-4-CF₃ | CH(CH₃)COOC₂H₅ |
| 834 | CH₃ | 4-OC₆H₄-4-CF₃ | CH₂C₆H₄-3-CF₃ |
| 835 | CH₃ | 4-OC₆H₄-4-CF₃ | CH₂C₆H₄-4-F |
| 836 | CH₃ | 4-OC₆H₄-4-CF₃ | CH₂C₆H₄-3-F |
| 837 | CH₃ | 4-OC₆H₄-4-CF₃ | CH₂C₆H₄-2-F |
| 838 | CH₃ | 4-OC₆H₄-4-CF₃ | C(=O)OC₂H₅ |
| 839 | CH₃ | 4-OC₆H₄-4-CF₃ | C(=O)NHCH₃ |
| 840 | CH₃ | 4-OC₆H₄-4-CF₃ | C(=O)C(=O)OC₂H₅ |
| 841 | CH₃ | 4-OC₆H₄-2-CF₃ | CH₃ |
| 842 | CH₃ | 4-OC₆H₄-2-CF₃ | C₂H₅ |
| 843 | CH₃ | 4-OC₆H₄-2-CF₃ | n-C₃H₇ |
| 844 | CH₃ | 4-OC₆H₄-2-CF₃ | i-C₃H₇ |
| 845 | CH₃ | 4-OC₆H₄-2-CF₃ | n-C₄H₉ |
| 846 | CH₃ | 4-OC₆H₄-2-CF₃ | n-C₆H₁₃ |
| 847 | CH₃ | 4-OC₆H₄-2-CF₃ | CH₂F |
| 848 | CH₃ | 4-OC₆H₄-2-CF₃ | CHF₂ |
| 849 | CH₃ | 4-OC₆H₄-2-CF₃ | CH₂CF₃ |
| 850 | CH₃ | 4-OC₆H₄-2-CF₃ | CH₂CH=CH₂ |
| 851 | CH₃ | 4-OC₆H₄-2-CF₃ | CH₂CH=CHCH₃ |
| 852 | CH₃ | 4-OC₆H₄-2-CF₃ | CH₂CH=C(CH₃)₂ |
| 853 | CH₃ | 4-OC₆H₄-2-CF₃ | CH₂CH=CHCl |
| 854 | CH₃ | 4-OC₆H₄-2-CF₃ | CH₂CH=CCl₂ |
| 855 | CH₃ | 4-OC₆H₄-2-CF₃ | CH₂C(CH₃)=CH₂ |
| 856 | CH₃ | 4-OC₆H₄-2-CF₃ | CH₂C≡CH |
| 857 | CH₃ | 4-OC₆H₄-2-CF₃ | CH₂Si(CH₃)₃ |
| 858 | CH₃ | 4-OC₆H₄-2-CF₃ | CH₂-c.propyl-2,2-Cl₂ |
| 859 | CH₃ | 4-OC₆H₄-2-CF₃ | CH₂CN |
| 860 | CH₃ | 4-OC₆H₄-2-CF₃ | CH₂COOC₂H₅ |
| 861 | CH₃ | 4-OC₆H₄-2-CF₃ | CH(CH₃)COOC₂H₅ |
| 862 | CH₃ | 4-OC₆H₄-2-CF₃ | CH₂C₆H₄-3-CF₃ |
| 863 | CH₃ | 4-OC₆H₄-2-CF₃ | CH₂C₆H₄-4-F |
| 864 | CH₃ | 4-OC₆H₄-2-CF₃ | CH₂C₆H₄-3-F |
| 865 | CH₃ | 4-OC₆H₄-2-CF₃ | CH₂C₆H₄-2-F |
| 866 | CH₃ | 4-OC₆H₄-2-CF₃ | C(=O)OC₂H₅ |
| 867 | CH₃ | 4-OC₆H₄-2-CF₃ | C(=O)NHCH₃ |
| 868 | CH₃ | 4-OC₆H₄-2-CF₃ | C(=O)C(=O)OC₂H₅ |
| 869 | CH₃ | 4-OCH₂C₆H₄-4-Cl | CH₃ |
| 870 | CH₃ | 4-OCH₂C₆H₄-4-Cl | C₂H₅ |
| 871 | CH₃ | 4-OCH₂C₆H₄-4-Cl | n-C₃H₇ |
| 872 | CH₃ | 4-OCH₂C₆H₄-4-Cl | i-C₃H₇ |
| 873 | CH₃ | 4-OCH₂C₆H₄-4-Cl | n-C₄H₉ |
| 874 | CH₃ | 4-OCH₂C₆H₄-4-Cl | n-C₆H₁₃ |
| 875 | CH₃ | 4-OCH₂C₆H₄-4-Cl | CH₂F |
| 876 | CH₃ | 4-OCH₂C₆H₄-4-Cl | CHF₂ |
| 877 | CH₃ | 4-OCH₂C₆H₄-4-Cl | CH₂CF₃ |
| 878 | CH₃ | 4-OCH₂C₆H₄-4-Cl | CH₂CH=CH₂ |
| 879 | CH₃ | 4-OCH₂C₆H₄-4-Cl | CH₂CH=CHCH₃ |
| 880 | CH₃ | 4-OCH₂C₆H₄-4-Cl | CH₂CH=C(CH₃)₂ |
| 881 | CH₃ | 4-OCH₂C₆H₄-4-Cl | CH₂CH=CHCl |
| 882 | CH₃ | 4-OCH₂C₆H₄-4-Cl | CH₂CH=CCl₂ |
| 883 | CH₃ | 4-OCH₂C₆H₄-4-Cl | CH₂C(CH₃)=CH₂ |
| 884 | CH₃ | 4-OCH₂C₆H₄-4-Cl | CH₂C≡CH |
| 885 | CH₃ | 4-OCH₂C₆H₄-4-Cl | CH₂Si(CH₃)₃ |
| 886 | CH₃ | 4-OCH₂C₆H₄-4-Cl | CH₂-c.propyl-2,2-Cl₂ |
| 887 | CH₃ | 4-OCH₂C₆H₄-4-Cl | CH₂CN |
| 888 | CH₃ | 4-OCH₂C₆H₄-4-Cl | CH₂COOC₂H₅ |
| 889 | CH₃ | 4-OCH₂C₆H₄-4-Cl | CH(CH₃)COOC₂H₅ |
| 890 | CH₃ | 4-OCH₂C₆H₄-4-Cl | CH₂C₆H₄-3-CF₃ |
| 891 | CH₃ | 4-OCH₂C₆H₄-4-Cl | CH₂C₆H₄-4-F |
| 892 | CH₃ | 4-OCH₂C₆H₄-4-Cl | CH₂C₆H₄-3-F |
| 893 | CH₃ | 4-OCH₂C₆H₄-4-Cl | CH₂C₆H₄-2-F |
| 894 | CH₃ | 4-OCH₂C₆H₄-4-Cl | C(=O)OC₂H₅ |
| 895 | CH₃ | 4-OCH₂C₆H₄-4-Cl | C(=O)NHCH₃ |
| 896 | CH₃ | 4-OCH₂C₆H₄-4-Cl | C(=O)C(=O)OC₂H₅ |
| 797 | CH₃ | 4-OCH₂C₆H₃-3,4-Cl₂ | CH₃ |
| 898 | CH₃ | 4-OCH₂C₆H₃-3,4-Cl₂ | C₂H₅ |
| 899 | CH₃ | 4-OCH₂C₆H₃-3,4-Cl₂ | n-C₃H₇ |
| 900 | CH₃ | 4-OCH₂C₆H₃-3,4-Cl₂ | i-C₃H₇ |
| 901 | CH₃ | 4-OCH₂C₆H₃-3,4-Cl₂ | n-C₄H₉ |
| 902 | CH₃ | 4-OCH₂C₆H₃-3,4-Cl₂ | n-C₆H₁₃ |
| 903 | CH₃ | 4-OCH₂C₆H₃-3,4-Cl₂ | CH₂F |
| 904 | CH₃ | 4-OCH₂C₆H₃-3,4-Cl₂ | CHF₂ |
| 905 | CH₃ | 4-OCH₂C₆H₃-3,4-Cl₂ | CH₂CF₃ |
| 906 | CH₃ | 4-OCH₂C₆H₃-3,4-Cl₂ | CH₂CH=CH₂ |
| 907 | CH₃ | 4-OCH₂C₆H₃-3,4-Cl₂ | CH₂CH=CHCH₃ |
| 908 | CH₃ | 4-OCH₂C₆H₃-3,4-Cl₂ | CH₂CH=C(CH₃)₂ |
| 909 | CH₃ | 4-OCH₂C₆H₃-3,4-Cl₂ | CH₂CH=CHCl |
| 910 | CH₃ | 4-OCH₂C₆H₃-3,4-Cl₂ | CH₂CH=CCl₂ |
| 911 | CH₃ | 4-OCH₂C₆H₃-3,4-Cl₂ | CH₂C(CH₃)=CH₂ |
| 912 | CH₃ | 4-OCH₂C₆H₃-3,4-Cl₂ | CH₂C≡CH |
| 913 | CH₃ | 4-OCH₂C₆H₃-3,4-Cl₂ | CH₂Si(CH₃)₃ |
| 914 | CH₃ | 4-OCH₂C₆H₃-3,4-Cl₂ | CH₂-c.propyl-2,2-Cl₂ |
| 915 | CH₃ | 4-OCH₂C₆H₃-3,4-Cl₂ | CH₂CN |
| 916 | CH₃ | 4-OCH₂C₆H₃-3,4-Cl₂ | CH₂COOC₂H₅ |
| 917 | CH₃ | 4-OCH₂C₆H₃-3,4-Cl₂ | CH(CH₃)COOC₂H₅ |
| 918 | CH₃ | 4-OCH₂C₆H₃-3,4-Cl₂ | CH₂C₆H₄-3-CF₃ |
| 919 | CH₃ | 4-OCH₂C₆H₃-3,4-Cl₂ | CH₂C₆H₄-4-F |
| 920 | CH₃ | 4-OCH₂C₆H₃-3,4-Cl₂ | CH₂C₆H₄-3-F |
| 921 | CH₃ | 4-OCH₂C₆H₃-3,4-Cl₂ | CH₂C₆H₄-2-F |
| 922 | CH₃ | 4-OCH₂C₆H₃-3,4-Cl₂ | C(=O)OC₂H₅ |
| 923 | CH₃ | 4-OCH₂C₆H₃-3,4-Cl₂ | C(=O)NHCH₃ |
| 924 | CH₃ | 4-OCH₂C₆H₃-3,4-Cl₂ | C(=O)C(=O)OC₂H₅ |

In Tables 2.1 and 2.2, the ¹³C-NMR data of the compounds 1-[4-(3-trifluoromethylphenylmethoxy)-phenyl]-1,2-propanedione 1-E-[methyloxime]-2-oxime and 1-[4-(3-trifluoromethylphenylmethoxy)-phenyl]-1,2-propanedione 1-Z-[methyloxime]-2-oxime (which was prepared by one of the known processes and from which the E/Z isomer mixture formed in the preparation was isolated) or, respectively, methyl 2-[[[(1-methyl-2-(4-(3-trifluoromethylphenylmethoxy)-phenyl)-2-E-[methoxyimino]ethylidene)amino]oxy]methyl)α-(methoxymethylene)-phenylacetate (compound A225 in Table 1) are shown. The similar chemical shifts of atoms 1 and 4 of compound A in Table 2.1 and those in Table 2.2 confirm the E configuration of the compounds of the formula I.

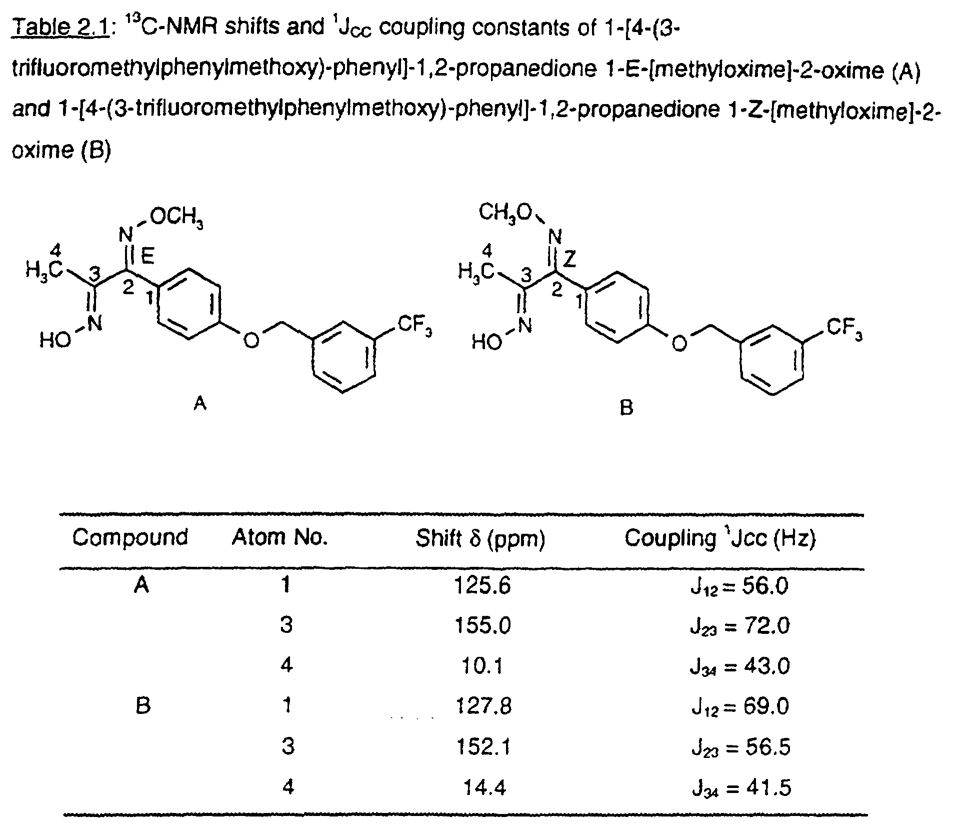

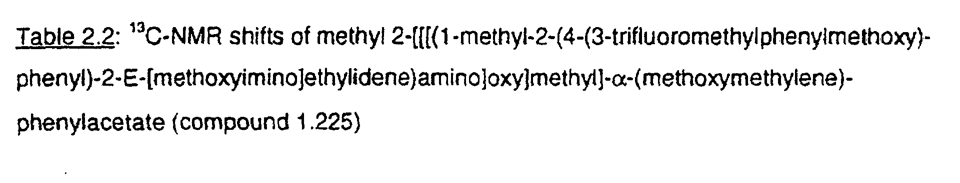

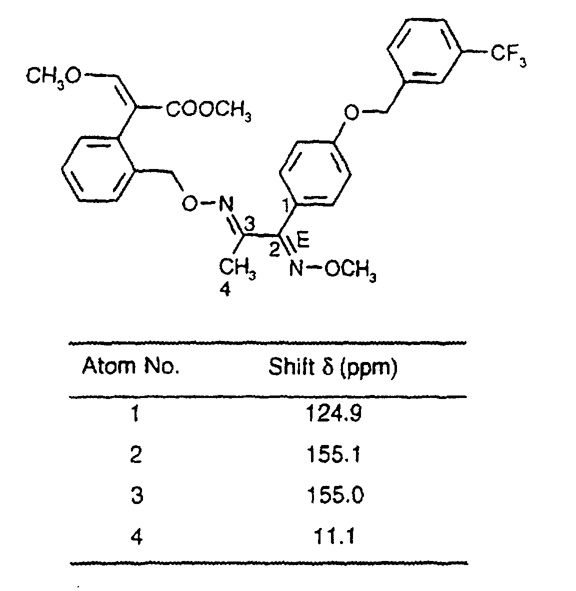

## Claims

1. A process for the preparation of a compound of the formula 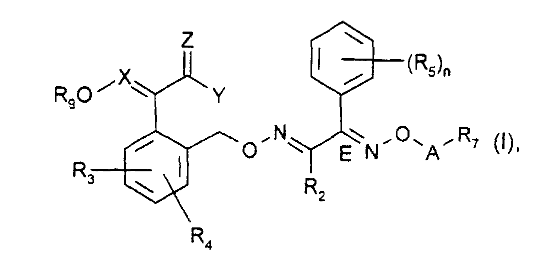 and, where appropriate, their tautomers, in each case in the free form or salt form, in which either
X is CH or N, Y is OR₁ and Z is O, or
X is N, Y is NHR₈ and Z is O, S or S(=O);
R₁ is C₁-C₄alkyl;
R₂ is H, C₁-C₄alkyl, halogeno-C₁-C₄alkyl, C₃-C₆cycloalkyl or C₁-C₄alkoxymethyl;
R₃ and R₄ independently of one another are H, C₁-C₄alkyl, C₁-C₄alkoxy, OH, CN, NO₂, a (C₁-C₄alkyl)₃-Si group, where the alkyl groups can be identical or different, halogen, (C₁-C₄alkyl)S(=O)ₘ, (halogeno-C₁-C₄alkyl)S(=O)ₘ, halogeno-C₁-C₄alkyl or halogeno-C₁-C₄alkoxy;
R₅ is C₁-C₆alkyl, halogeno-C₁-C₆alkyl, C₁-C₆alkoxy, halogeno-C₁-C₆alkoxy, C₁-C₆-alkylthio, halogen-C₁-C₆alkylthio, C₁-C₆alkylsulfinyl, halogeno-C₁-C₆-alkylsulfinyl, C₁-C₆alkylsulfonyl, halogeno-C₁-C₆alkylsulfonyl, C₁-C₆alkoxy-C₁-C₆alkyl, halogeno-C₁-C₆alkoxy-C₁-C₆alkyl, C₁-C₆alkylthio-C₁-C₆alkyl, halogeno-C₁-C₆alkylthio-C₁-C₆alkyl, C₁-C₆alkylsulfinyl-C₁-C₆alkyl, halogeno-C₁-C₆-alkylsulfinyl-C₁-C₆alkyl, C₁-C₆-alkylsulfonyl-C₁-C₆alkyl, halogeno-C₁-C₆-alkylsulfonyl-C₁-C₆alkyl, C₁-C₆-alkylcarbonyl halogeno-C₁-C₆-alkylcarbonyl, C₁-C₆-alkoxycarbonyl, halogeno-C₁-C₆-alkoxycarbonyl, C₁-C₆-alkylaminocarbonyl, C₁-C₄-alkoxyiminomethyl; di(C₁-C₆alkyl)-aminocarbonyl, where the alkyl groups can be identical or different; C₁-C₆-alkylaminothiocarbonyl; di(C₁-C₆alkyl)aminothiocarbonyl, where the alkyl groups can be identical or different; C₁-C₆-alkylamino, di(C₁-C₆alkyl)-amino, where the alkyl groups can be identical or different; halogen, NO₂, CN, SF₅, thioamido, thiocyanatomethyl; an unsubstituted or mono- to tetrasubstituted C₁-C₄alkylenedioxy group, where the substituents are selected from the group consisting of C₁-C₄alkyl and halogen; or QR₆, where, if n is greater than 1, the radicals R₅ can be identical or different;
R₆ is C₂-C₆alkenyl or C₂-C₆ alkynyl, which are unsubstituted or substituted by 1 to 3 halogen atoms; (C₁-C₄alkyl)₃Si, where the alkyl groups can be identical or different; CN; or an unsubstituted or mono- to pentasubstituted C₃-C₆cycloalkyl, aryl or heterocyclyl group, where the substituents are selected from the group consisting of halogen, C₁-C₆alkyl, halogeno-C₁-C₆alkyl, C₁-C₆alkoxy, halogeno-C₁-C₆alkoxy, phenoxy, naphthoxy and CN;
A either is a direct bond, C₁-C₁₀alkylene, -C(=O)-,-C(=S)- or halogeno-C₁-C₁₀alkylene and R₇ is a radical R₁₀,
or is C₁-C₁₀alkylene, -C(=O)-,-C(=S)- or halogeno-C₁-C₁₀alkylene and
R₇ is OR₁₀, N(R₁₀)₂, where the radicals R₁₀ can be identical or different, or -S(=O)_{q}R₁₀;
R₈ is H or C₁-C₄alkyl;
R₉ is methyl, fluoromethyl or difluoromethyl;
R₁₀ is H; an unsubstituted or substituted C₁-C₆alkyl, C₂-C₆alkenyl or C₂-C₆alkynyl group,
where the substituents are selected from the group consisting of halogen; (C₁-C₄alkyl)₃Si, where the alkyl groups can be identical or different; C₃-C₆cyclo-alkyl, which is unsubstituted or substituted by halogen; C₁-C₆alkoxycarbonyl, which is unsubstituted or substituted by halogen; unsubstituted or substituted aryl, where the substituents are selected from the group consisting of halogen, halogeno-C₁-C₄alkyl and CN; a (C₁-C₄alkyl)₃Si group, where the alkyl groups can be identical or different; C₃-C₆cycloalkyl, which is unsubstituted or substituted by halogen; C₁-C₆alkoxycarbonyl which is unsubstituted or substituted by halogen; or an unsubstituted or substituted aryl or heterocyclyl group, where the substituents are selected from the group consisting of halogen and halogeno-C₁-C₄alkyl;
Q is a direct bond, C₁-C₈alkylene, C₂-C₆alkenylene, C₂-C₆alkynylene, O, O(C₁-C₆alkylene), (C₁-C₆alkylene)O, S(=O)ₚ, S(=O)ₚ(C₁-C₆alkylene) or (C₁-C₆alkylene)S(=O)ₚ;
m is 0, 1 or 2;
n is 0, 1, 2, 3, 4 or 5;
p is 0, 1 or 2; and
q is 0, 1 or 2,
and the C=N double bond marked with E has the E-configuration, which comprises
c) reacting a compound of the formula 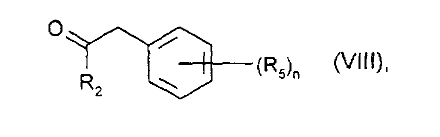 in which R₂, R₅ and n are as defined for formula (I), or a possible tautomer thereof, in each case in the free form or in salt form with a C₁-C₆alkyl nitrite in order to obtain a compound of the formula 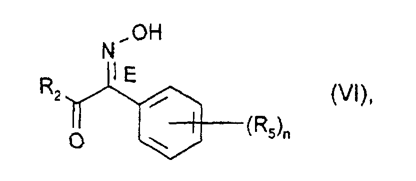 in which R₂, R₅ and n are as defined for formula (I) and the C=N double bond marked with E has the E-configuration, or a possible tautomer thereof, in each case in the free form or in salt form;
b1) converting the said compound of the formula (VI) into a compound of the formula 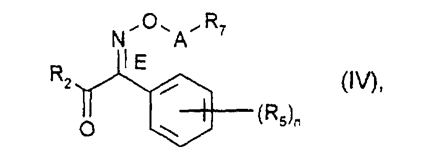 in which A, R₂, R₅, R₇ and n are as defined for formula (I) and the C=N double bond marked with E has the E-configuration, or a possible tautomer thereof, in each case in the free form or in the salt form, by reacting the compound of the formula (VI) with a compound of the formula
R₇-A-X₂ (VII),
in which A and R₇ are as defined for formula (I) and X₂ is a leaving group, and, in order to obtain a compound of the formula (I), *either*
a2) reacting a compound of the formula (IV) with a compound of the formula 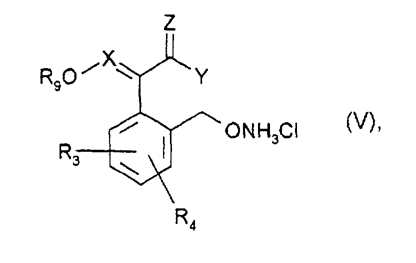 in which X, Y, Z, R₃, R₄ and R₉ are as defined for formula (I), or, if appropriate, a tautomer thereof, in each case in the free form or in salt form; *or*
b2) converting the compound of the formula (IV) into a compound of the formula 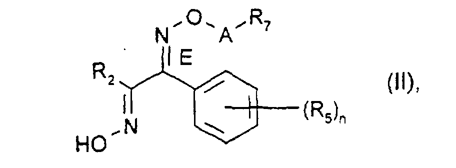 in which A, R₂, R₅, R₇ and n are as defined for formula (I) and the C=N double bond marked with E has the E-configuration, or a possible tautomer thereof, in each case in the free form or in salt form, by reacting the compound of the formula (IV) with hydroxylamine or a salt thereof; and
a1) reacting a compound of the formula (II) with a compound of the formula 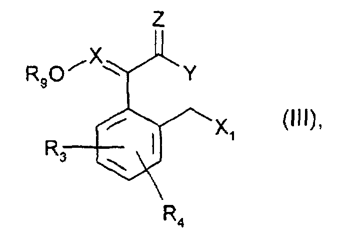 in which X, Y, Z, R₃, R₄ and R₉ are as defined for formula (I) and X₁ is a leaving group, or a tautomer thereof, in each case in the free from or in salt form.

2. A process according to claim 1 for the preparation of a compound of the formula (I), which comprises reacting a compound of the formula (II) with a compound of the formula (III).

3. A process according to claim 2, wherein a compound of the formula (III) in which X₁ is halogen is used.

4. A process according to claim 1 for the preparation of a compound of the formula (I), which comprises reacting the compound of the formula (IV), with a compound of the formula (V).

5. A process according to claim 1 for the preparation of a compound of the formula (I), which comprises reacting the compound of the formula (VI), with a compound of the formula (VII), and either reacting the compound thus obtainable, of the formula (IV), according to the process according to claim 4, or reacting it with hydroxylamine or a salt thereof, if appropriate in the presence of a basic or acid catalyst, and further reacting the compound thus obtainable, of the formula (II), according to the process according to claim 2.

6. A process according to claim 5, wherein a compound of the formula (VII) in which X₂ is halogen is used.

7. A process according to claim 5, wherein a compound of the formula (VII) in which X₂ is chlorine is used.

8. A process according to claim 1 for the preparation of a compound of the formula (I), which comprises reacting the compound of the formula (VIII), with a C₁-C₆ alkyl nitrite and further reacting the compound thus obtainable, of the formula (VI), according to the process according to claim 5.

9. A process for the preparation of a compound of the formula 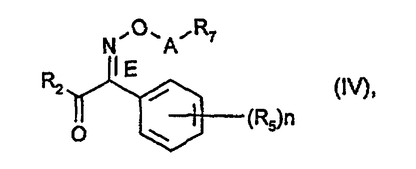 in which A, R₂, R₅, R₇ and n are as defined in claim 1 for formula (I) and the C=N double bond marked with E has the E-configuration, which comprises
c) reacting a compound of the formula (VIII) as defined in claim 1 with a C₁-C₆alkyl nitrite in order to obtain a compound of the formula (VI) as defined in claim 1; and
b1) converting the said compound of the formula (VI) into a compound of the formula (IV), as defined above by reacting the compound of the formula (VI) with a compound of the formula (VII), as defined in claim 1.

10. A process according to claim 9, wherein a compound of the formula (VII) in which X₂ is halogen is used.

11. A process according to claim 10, wherein a compound of the formula (VII) in which X₂ is chlorine is used.

12. A process for the preparation of a compound of the formula 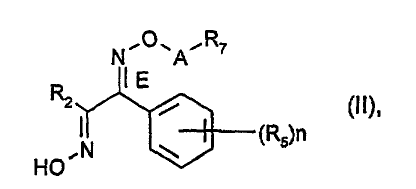 in which A, R₂, R₅, R₇ and n are as defined in claim 1 for formula (I) and the C=N double bond marked with E has the E-configuration, which comprises
c) reacting a compound of the formula (VIII) as defined in claim 1, with a C₁-C₆alkyl nitrite in order to obtain a compound of the formula (VI), as defined in claim 1;
b1) converting the said compound of the formula (VI) into a compound of the formula (IV), as defined in claim 1, by reacting the compound of the formula (VI) with a compound of the formula (VII), as defined in claim 1; and
b2) converting the compound of the formula (IV) into a compound of the formula (II) by reacting the compound of the formula (IV) with hydroxylamine or a salt thereof.

13. A process according to claim 12, wherein the reaction of step b2) is carried out with hydroxylamine hydrochloride.

14. A process for the preparation of a compound of the formula 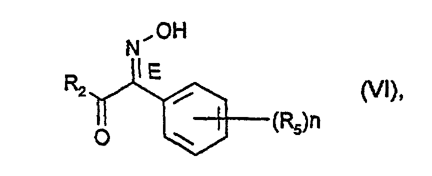 in which R₂, R₅ and n are as defined in claim 1 for formula (I) and the C=N double bond marked with E has the E-configuration, which comprises reacting the compound of the formula (VIII), as defined in claim 1, with a C₁-C₆alkyl nitrite.

15. A process according to claim 14, wherein the reaction is carried out in the presence of a base.

16. A process according to claim 15, wherein the reaction is carried out in the presence of a base selected from the group consisting of alkali metal and alkaline earth metal hydroxides, hydrides, amides, alkanolates, acetates, carbonates, dialkylamide and alkylsilylamides.

17. A process according to claim 16, wherein the base is sodium methanolate.

18. A process according to claim 14, wherein the reaction is carried out in the presence of a solvent or diluent or of a mixture thereof.

19. A process according to claim 18, wherein the solvent is selected from the group consisting of methanol, ethanol, propanol, isopropanol, butanol, ethylene glycol and glycerol.

20. A process according to claim 19, wherein the reaction is carried out in methanol.

## Patentansprüche

1. Verfahren zur Herstellung einer Verbindung der Formel: 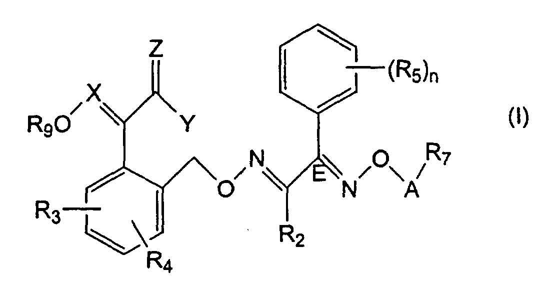 und gegebenenfalls von deren Tautomeren, jeweils in der freien Form oder der Salz-Form, worin gilt:
entweder sind
X CH oder N, Y OR₁ und Z O
oder sind
X N, Y NHR₈ und Z O, S oder S(=O);
R₁ ist C₁₋₄-Alkyl;
R₂ ist H, C₁₋₄-Alkyl, Halogen-C₁₋₄-alkyl, C₃₋₆-Cycloalkyl oder C₁₋₄-Alkoximethyl;
R₃ und R₄ sind, unabhängig voneinander, H, C₁₋₄-Alkyl, C₁₋₄-Alkoxi, OH, CN, NO₂, eine (C₁₋₄-Alkyl)₃Si-Gruppe, worin die Alkylgruppen, gleich oder verschieden, Halogen, (C₁₋₄-Alkyl)S(=O)ₘ, (Halogen-C₁₋₄-alkyl)S(=O)ₘ, Halogen-C₁₋₄-alkyl oder Halogen-C₁₋₄-alkoxi sein können;
R₅ ist C₁₋₆-Alkyl, Halogen-C₁₋₆-alkyl, C₁₋₆-Alkoxi, Halogen-C₁₋₆-alkoxi, C₁₋₆-Alkylthio, Halogen-C₁₋₆-alkylthio, C₁₋₆-Alkylsulfinyl, Halogen-C₁₋₆-alkylsulfinyl, C₁₋₆-Alkylsulfonyl, Halogen-C₁₋₆-alkylsulfonyl, C₁₋₆-Alkoxi-C₁₋₆-alkyl, Halogen-C₁₋₆-alkoxi-C₁₋₆-alkyl, C₁₋₆-Alkylthio-C₁₋₆-alkyl, Halogen-C₁₋₆-alkylthio-C₁₋₆-alkyl, C₁₋₆-Alkylsulfinyl-C₁₋₆-alkyl, Halogen-C₁₋₆-alkylsulfinyl-C₁₋₆-alkyl, C₁₋₆-Alkylsulfonyl-C₁₋₆-alkyl, Halogen-C₁₋₆-alkylsulfonyl, C₁₋₆-Alkyl, C₁₋₆-Alkylcarbonyl, Halogen-C₁₋₆-alkylcarbonyl, C₁₋₆-Alkoxicarbonyl, Halogen-C₁₋₆-alkoxicarbonyl, C₁₋₆-Alkylaminocarbonyl, C₁₋₄-Alkoximinomethyl, Di(C₁₋₆-alkyl)aminocarbonyl, worin die Alkylgruppen gleich oder verschieden sein können, C₁₋₆-Alkylaminothiocarbonyl, Di(C₁₋₆--alkyl)aminothiocarbonyl, worin die Alkylgruppen gleich oder verschieden sein können, C₁₋₆-Alkylamino, Di(C₁₋₆-alkyl)amino, worin die Alkylgruppen gleich oder verschieden sein können, Halogen, NO₂, CN, SF₅, Thioamido, Thiocyanatomethyl, eine unsubstituierte oder mono- bis tetrasubstituierte C₁₋₄-Alkylendioxi-Gruppe, worin die Substituenten aus der Gruppe ausgewählt sind, bestehend aus C₁₋₄-Alkyl und Halogen, oder QR₆,
worin, falls n größer als 1 ist, die Reste R₅ gleich oder verschieden sein können;
R₆ ist C₂₋₆-Alkenyl oder C₂₋₆-Alkinyl, die unsubstituiert oder mit 1 bis 3 Halogenatomen substituiert sind, (C₁₋₄-Alkyl)₃Si, worin die Alkylgruppen gleich oder verschieden sein können, CN, oder eine unsubstituierte oder mono- bis pentasubstituierte C₃₋₆-Cycloalkyl-, Aryl- oder Heterocyclylgruppe, worin die Substituenten aus der Gruppe ausgewählt sind, bestehend aus Halogen, C₁₋₆-Alkyl, Halogen-C₁₋₆-alkyl, C₁₋₆-Alkoxi, Halogen-C₁₋₆-alkoxi, Phenoxi, Naphthoxi und aus CN;
A ist entweder eine direkte Bindung, C₁₋₁₀-Alkylen, -C(=O)-, -C(=S)- oder Halogen-C₁₋₁₀-alkylen und R₇ ist ein Rest R₁₀ oder ist A C₁₋₁₀-Alkylen, -C(=O)-, -C(=S)- oder Halogen-C₁₋ ₁₀-Alkylen und R₇ ist OR₁₀, N(R₁₀)₂ oder -S(=O)_{q}R₁₀, worin die Reste R₁₀ gleich oder verschieden sein können;
R₈ ist H oder C₁₋₄-Alkyl;
R₉ ist Methyl, Fluormethyl oder Difluormethyl;
R₁₀ ist H, eine unsubstituierte oder substituierte C₁₋₆-Alkyl-, C₂₋₆-Alkenyl- oder C₂₋₆-Alkinylgruppe, worin die Substituenten aus der Gruppe ausgewählt sind, bestehend aus Halogen, (C₁₋₄-Alkyl)₃Si, worin die Alkylgruppen gleich oder verschieden sein können, C₃₋₆-Cycloalkyl, das unsubstituiert oder mit Halogen substituiert ist, C₁₋₆-Alkoxicarbonyl, das unsubstituiert oder mit Halogen substituiert ist, und aus unsubstituiertem oder substituiertem Aryl, worin die Substituenten aus der Gruppe ausgewählt sind, bestehend aus Halogen, Halogen-C₁₋₄-alkyl und aus CN, eine (C₁₋₄-Alkyl)₃-Si-Gruppe, worin die Alkylgruppen gleich oder verschieden sein können, C₃₋₆-Cycloalkyl, das unsubstituiert oder mit Halogen substituiert ist, C₁₋₆-Alkoxicarbonyl, das unsubstituiert oder mit Halogen substituiert ist, oder eine unsubstituierte oder substituierte Aryl- oder Heterocyclylgruppe, worin die Substituenten aus der Gruppe ausgewählt sind, bestehend aus Halogen und Halogen-C₁₋₄-alkyl;
Q ist eine direkte Bindung, C₁₋₈-Alkylen, C₂₋₆-Alkenylen, C₂₋₆--Alkinylen, O, O(C₁₋₆-Alkylen), (C₁₋₆-Alkylen)O, S(=O)ₚ, S(=O)ₚ(C₁₋₆-Alkylen) oder (C₁₋₆-Alkylen) S (=O)ₚ;
m ist 0, 1 oder 2;
n ist 0, 1, 2, 3, 4 oder 5;
p ist 0, 1 oder 2; und
q ist 0, 1 oder 2;
und die C=N-Doppelbindung, die mit E markiert ist, weist die E-Konfiguration auf, wobei man
c) eine Verbindung der Formel: 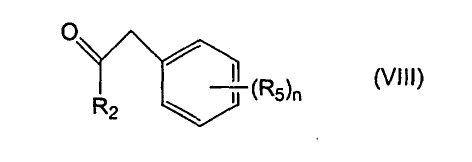 worin R₂, R₅ und n wie für Formel (I) definiert sind, oder ein mögliches Tautomer davon, jeweils in der freien Form oder in Salz-Form, mit einem C₁₋₆-Alkylnitrit zur Reaktion bringt, um eine Verbindung der Formel: 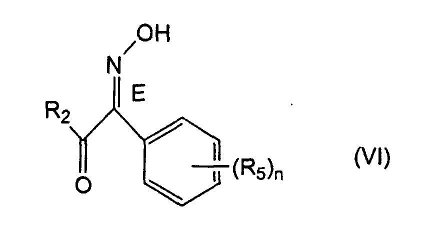 worin R₂, R₅ und n wie für Formel (I) definiert sind und die mit E markierte C=N-Doppelbindung die E-Konfiguration aufweist, oder ein mögliches Tautomer davon, jeweils in der freien Form oder in der Salz-Form, zu erhalten,
b1) man die genannte Verbindung der Formel (VI) in eine Verbindung der Formel: 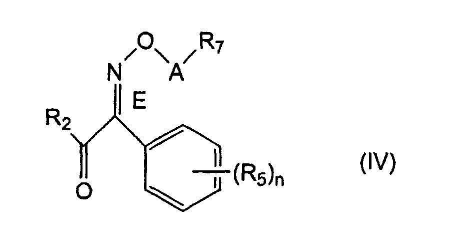 worin A, R₂, R₅, R₇ und n wie für Formel (I) definiert sind und die mit E markierte C=N-Doppelbindung die E-Konfiguration aufweist, oder in ein mögliches Tautomer davon, jeweils in die freie Form oder in die Salz-Form, überführt, indem man die Verbindung der Formel (VI) mit einer Verbindung der Formel zur Reaktion bringt:
**R**_{**7**}**-A-X**_{**2**} **(VII)**
worin A und R₇ wie für Formel (I) definiert und X₂ eine Abgangsgruppe sind, und wobei man zum Erhalt einer Verbindung der Formel (I) entweder
a2) eine Verbindung der Formel (IV) mit einer Verbindung der Formel: 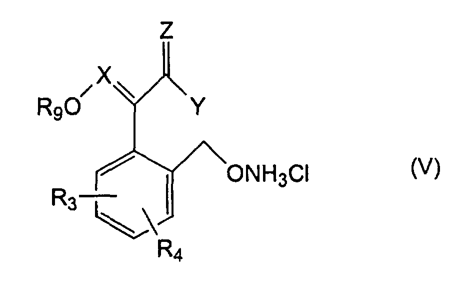 worin X, Y, Z, R₃, R₄ und R₉ wie für Formel (I) definiert sind, oder gegebenenfalls ein Tautomer davon, jeweils in der freien Form oder in Salz-Form, zur Reaktion bringt oder man
b2) die Verbindung der Formel (IV) in eine Verbindung der Formel: 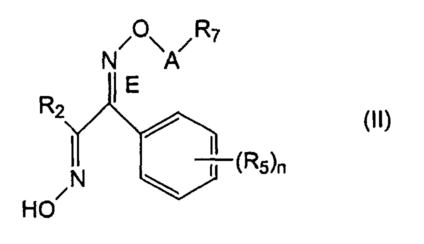 worin A, R₂, R₅, R₇ und n wie für Formel (I) definiert sind und die mit E markierte C=N-Doppelbindung die E-Konfiguration aufweist, oder in ein mögliches Tautomer davon, jeweils in die freie Form oder in die Salz-Form, überführt, indem man die Verbindung der Formel (IV) mit Hydroxylamin oder einem Salz davon zur Reaktion bringt, und man
a1) eine Verbindung der Formel (II) mit einer Verbindung der Formel: 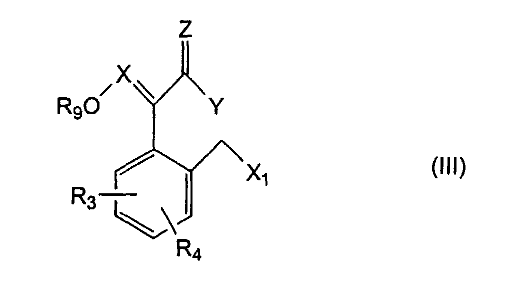 worin X, Y, Z, R₃, R₄ und R₉ wie für Formel (I) definiert und X₁ eine Abgangsgruppe sind, oder dessen Tautomer, jeweils in der freien Form oder in Salz-Form, zur Reaktion bringt.

2. Verfahren gemäß Anspruch 1 zur Herstellung einer Verbindung der Formel (I), wobei man eine Verbindung der Formel (II) mit einer Verbindung der Formel (III) zur Reaktion bringt.

3. Verfahren gemäß Anspruch 2, wobei eine Verbindung der Formel (III), worin X₁ Halogen ist, verwendet wird.

4. Verfahren gemäß Anspruch 1 zur Herstellung einer Verbindung der Formel (I), wobei man die Verbindung der Formel (IV) mit einer Verbindung der Formel (V) zur Reaktion bringt.

5. Verfahren gemäß Anspruch 1 zur Herstellung einer Verbindung der Formel I, wobei man die Verbindung der Formel (VI) mit einer Verbindung der Formel (VII) zur Reaktion bringt und man entweder die so erhältliche Verbindung der Formel (IV) gemäß dem Verfahren gemäß Anspruch 4 oder mit Hydroxylamin oder einem Salz davon, gegebenenfalls in der Gegenwart eines basischen oder sauren Katalysators, zur Reaktion bringt, worauf man die so erhältliche Verbindung der Formel (II) gemäß dem Verfahren gemäß Anspruch 2 weiterreagieren lässt.

6. Verfahren gemäß Anspruch 5, wobei eine Verbindung der Formel (VII), worin X₂ Halogen ist, verwendet wird.

7. Verfahren gemäß Anspruch 5, wobei eine Verbindung der Formel (VII), worin X₂ Chlor ist, verwendet wird.

8. Verfahren gemäß Anspruch 1 zur Herstellung einer Verbindung der Formel (I), wobei man die Verbindung der Formel (VIII) mit einem C₁₋₆-Alkylnitrit zur Reaktion bringt und die so erhältliche Verbindung der Formel (VI) gemäß dem Verfahren gemäß Anspruch 5 weiterreagieren lässt.

9. Verfahren zur Herstellung einer Verbindung der Formel : 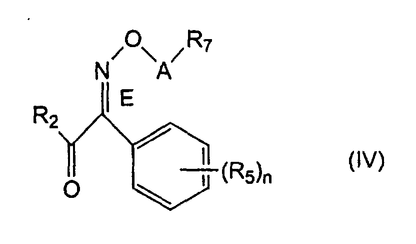 worin A, R₂, R₅, R₇ und n wie in Anspruch 1 für Formel (I) definiert sind und die mit E markierte C=N-Doppelbindung die E-Konfiguration aufweist, wobei man
c) eine Verbindung der in Anspruch 1 definierten Formel (VIII) mit einem C₁₋₆-Alkylnitrit zur Reaktion bringt, um eine Verbindung der in Anspruch 1 definierten Formel (VI) zu erhalten, und man
b1) die genannte Verbindung der Formel (VI) in eine Verbindung der oben definierten Formel (IV) durch Reaktion der Verbindung der Formel (VI) mit einer Verbindung der in Anspruch 1 definierten Formel (VII) überführt.

10. Verfahren gemäß Anspruch 9, wobei eine Verbindung der Formel (VII), worin X₂ Halogen ist, verwendet wird.

11. Verfahren gemäß Anspruch 10, wobei eine Verbindung der Formel (VII), worin X₂ Chlor ist, verwendet wird.

12. Verfahren zur Herstellung einer Verbindung der Formel: 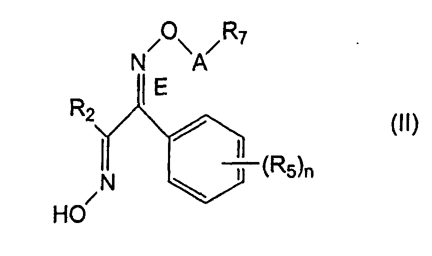 worin A, R₂, R₅, R₇ und n wie in Anspruch 1 für Formel (I) definiert sind und die mit E markierte C=N-Doppelbindung die E-Konfiguration aufweist, wobei man
c) eine Verbindung der in Anspruch 1 definierten Formel (VIII) mit einem C₁₋₆-Alkylnitrit zur Reaktion bringt, um eine Verbindung der in Anspruch 1 definierten Formel (VI) zu erhalten,
b1) die genannte Verbindung der Formel (VI) in eine Verbindung der in Anspruch 1 definierten Formel (IV) durch Reaktion der Verbindung der Formel (VI) mit einer Verbindung der in Anspruch 1 definierten Formel (VII) überführt und man
b2) die Verbindung der Formel (IV) in eine Verbindung der Formel (II) durch Reaktion der Verbindung der Formel (IV) mit Hydroxylamin oder einem Salz davon überführt.

13. Verfahren gemäß Anspruch 12, wobei die Reaktion von Stufe b2) mit Hydroxylamin-Hydrochlorid durchgeführt wird.

14. Verfahren zur Herstellung einer Verbindung der Formel: 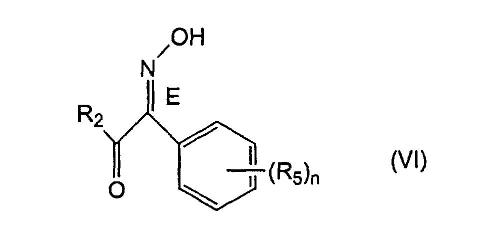 worin R₂, R₅ und n wie in Anspruch 1 für Formel (I) definiert sind und die mit E markierte C=N-Doppelbindung die E-Konfiguration aufweist, wobei man die Verbindung der in Anspruch 1 definierten Formel (VIII) mit einem C₁₋₆-Alkylnitrit zur Reaktion bringt.

15. Verfahren gemäß Anspruch 14, wobei die Reaktion in der Gegenwart einer Base durchgeführt wird.

16. Verfahren gemäß Anspruch 15, wobei die Reaktion in der Gegenwart einer Base, ausgewählt aus der Gruppe, bestehend aus Alkali- und Erdalkalimetallhydroxiden, -hydriden, -amiden, -alkanolaten, -acetaten, -carbonaten, -dialkylamiden und aus -alkylsilylamiden, durchgeführt wird.

17. Verfahren gemäß Anspruch 16, wobei die Base Natriummethanolat ist.

18. Verfahren gemäß Anspruch 14, wobei die Reaktion in der Gegenwart eines Lösungs- oder Verdünnungsmittels oder einer Mischung davon durchgeführt wird.

19. Verfahren gemäß Anspruch 18, worin das Lösungsmittel aus der Gruppe, bestehend aus Methanol, Ethanol, Propanol, Isopropanol Butanol, Ethylenglycol und aus Glycerin, ausgewählt ist.

20. Verfahren gemäß Anspruch 19, wobei die Reaktion in Methanol durchgeführt wird.

## Revendications

1. Procédé de préparation d'un composé de formule 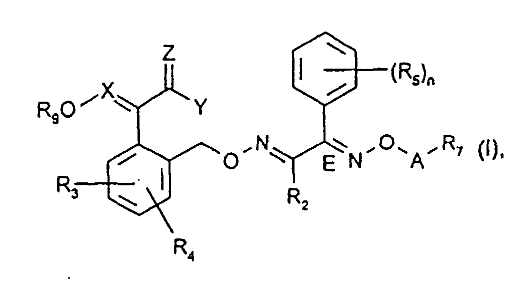 et, selon le cas, de leurs tautomères, dans chaque cas sous la forme libre ou sous la forme de sel,
formule dans laquelle
X représente CH ou N, Y est un groupe OR₁ et Z représente O, ou bien
X représente N, Y est un groupe NHR₈ et Z représente O, S ou S(=O) ;
R₁ est un groupe alkyle en C₁ à C₄ ;
R₂ représente H, un groupe alkyle en C₁ à C₄, halogénalkyle en C₁ à C₄, cycloalkyle en C₃ à C₆ ou (alkoxy en C₁ à C₄)méthyle ;
R₃ et R₄ représentent, indépendamment l'un de l'autre, H, un groupe alkyle en C₁ à C₄, alkoxy en C₁ à C₄, OH, CN, NO₂, un groupe (alkyle en C₁ à C₄)₃-Si dont les groupes alkyle peuvent être identiques ou différents, un halogène, un groupe (alkyle en C₁ à C₄)S(=O)ₘ, (halogénalkyle en C₁ à C₄) S(=O)ₘ, halogénalkyle en C₁ à C₄ ou halogénalkoxy en C₁ à C₄ ;
R₅ est un groupe alkyle en C₁ à C₆, halogénalkyle en C₁ à C₆, alkoxy en C₁ à C₆, halogénalkoxy en C₁ à C₆, alkylthio en C₁ à C₆, halogénalkylthio en C₁ à C₆, alkylsulfinyle en C₁ à C₆, halogénalkylsulfinyle en C₁ à C₆, alkylsulfonyle en C₁ à C₆, halogénalkylsulfonyle en C₁ à C₆, (alkoxy en C₁ à C₆)-(alkyle en C₁ à C₆), (halogénalkoxy en C₁ à C₆)-(alkyle en C₁ à C₆), (alkylthio en C₁ à C₆)-(alkyle en C₁ à C₆), (halogénalkylthio en C₁ à C₆)-(alkyle en C₁ à C₆), (alkylsulfinyle en C₁ à C₆)-(alkyle en C₁ à C₆), (halogénalkylsulfinyle en C₁ à C₆)-(alkyle en C₁ à C₆), (alkylsulfonyle en C₁ à C₆)-(alkyle en C₁ à C₆), (halogénalkylsulfonyle en C₁ à C₆)-(alkyle en C₁ à C₆), (alkyle en C₁ à C₆)carbonyle, (halogénalkyle en C₁ à C₆)carbonyle, (alkoxy en C₁ à C₆)carbonyle, (halogénalkoxy en C₁ à C₆)carbonyle, (alkylamino en C₁ à C₆)carbonyle, (alkoxyimino en C₁ à C₄)méthyle ; di(alkyle en C₁ à C₆)aminocarbonyle dont les groupes alkyle peuvent être identiques ou différents, (alkyle en C₁ à C₆)aminothiocarbenyle ; di(alkyle en C₁ à C₆)aminothiocarbonyle dont les groupes alkyle peuvent être identiques ou différents ; alkylamino en C₁ à C₆, di(alkyle en C₁ à C₆)amino dont les groupes alkyle peuvent étre identiques ou différents ; un halogène, un groupe NO₂, CN, SF₅, thio-amido, thiocyanatométhyle ; un groupe alkylènedioxy en C₁ à C₄ non substitué ou monosubstitué à tétrasubstitué, dont les substituants sont choisis dans le groupe consistant en alkyle en C₁ à C₄ et halogène ; ou un groupe QR₆ pour lequel, si n est supérieur à 1, les radicaux R₅ peuvent être identiques ou différents ;
R₆ est un groupe alcényle en C₂ à C₆ ou un groupe alcynyle en C₂ à C₆, qui sont non substitués ou qui sont substitués par 1 à 3 atomes d'halogènes ; un groupe (alkyle en C₁ à C₄)₃Si dans lequel les groupes alkyle peuvent être identiques ou différents ; un groupe CN ; ou un groupe cycloalkyle en C₃ à C₆, aryle ou hétérocyclyle non substitué ou mono- à pentasubstitué, dont les substituants sont choisis dans le groupe consistant en halogène, alkyle en C₁ à C₆, halogénalkyle en C₁ à C₆, alkoxy en C₁ à C₆, halogénalkoxy en C₁ à C₆, phénoxy, naphtoxy et CN ;
A représente une liaison simple, un groupe alkylène en C₁ à C₁₀, -C(=O)-, -C(=S)- ou halogénalkylène en C₁ à C₁₀ et
R₇ est un groupe R₁₀,
ou bien A est un groupe alkylène en C₁ à C₁₀, -C(=O)-, -C(=S)- ou halogénalkylène en C₁ à C₁₀ et
R₇ est un groupe OR₁₀, N(R₁₀)₂ dont les radicaux R₁₀ peuvent être identiques ou différents, ou bien un groupe -S(=O)_{q}R₁₀ ;
R₈ représente H ou un groupe alkyle en C₁ à C₄ ;
R₉ est un groupe méthyle, fluorométhyle ou difluorométhyle ;
R₁₀ représente H ; un groupe alkyle en C₁ à C₆, alcényle en C₂ à C₆ ou alcynyle en C₂ à C₆ non substitué ou substitué, dont les substituants sont choisis dans le groupe consistant en halogène ; (alkyle en C₁ à C₄)₃Si dont les groupes alkyle peuvent être identiques ou différents ; cycloalkyle en C₃ à C₆ non substitué ou substitué par un halogène ; (alkoxy en C₁ à C₆)-carbonyle non substitué ousubstitué par un halogène ; aryle non substitué ou substitué, dont les substituants sont choisis dans le groupe consistant en halogène, halogénalkyle en C₁ à C₄ et CN ; un groupe (alkyle en C₁ à C₄)₃Si dont les groupes alkyle peuvent être identiques ou différents ; un groupe cycloalkyle en C₃ à C₆ qui est non substitué ou substitué par un halogène ; un groupe (alkoxy en C₁ à C₆)carbonyle qui est non substitué ou substitué par un halogène ; ou un groupe aryle ou hétérocyclyle non substitué ou substitué dont les substituants sont choisis dans le groupe consistant en halogène et halogénalkyle en C₁ à C₄ ;
Q est une liaison simple, un groupe alkylène en C₁ à C₈, alcénylène en C₂ à C₆, (alcynylène en C₂ à C₆)O, un groupe (alkylène en C₁ à C₆) O, S- (=O)ₚ, S (=O)ₚ (alkylène en C₁ à C₆) ou (alkylène en C₁ à C₆) S (=O)ₚ ;
m a la valeur 0, 1 ou 2 ;
n a la valeur 0, 1, 2, 3, 4 ou 5 ;
p a la valeur 0,1 ou 2 ; et
q a la valeur 0, 1 ou 2,
et la double liaison C=N marquée avec la lettre E a la configuration E, qui comprend
c) la réaction d'un composé de formule 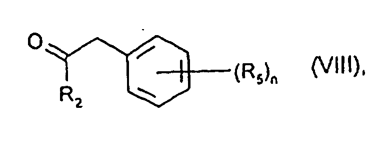 dans laquelle R₂, R₅ et n sont tels que définis pour la formule (I), ou d'un tautomère possible de ce composé, dans chaque cas sous la forme libre ou sous la forme de sel avec un nitrite d'alkyle en C₁ à C₆ en vue d'obtenir un composé de formule 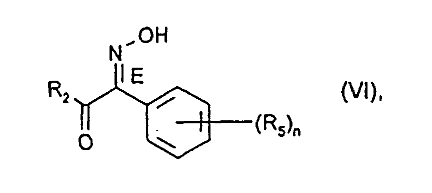 dans laquelle R₂, R₅ et n sont tels que définis pour la formule (I) et la double liaison C=N marquée de la lettre E a la configuration E, ou d'un tautomère possible de ce composé, dans chaque cas sous la forme libre ou sous la forme d'un sel
b1) la conversion du composé de formule (VI) en un composé de formule 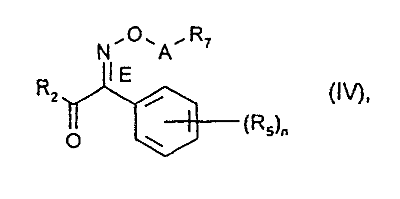 dans laquelle A, R₂, R₅, R₇ et n sont tels que définis pour la formule (I) et la double liaison C=N marquée de la lettre E a la configuration E, ou d'un tautomère possible de ce composé, dans chaque cas sous la forme libre ou sous la forme d'un sel, par réaction du composé de formule (VI) avec un composé de formule
R₇-A-X₂ (VII)
dans laquelle A et R₇ sont tels que définis pour la formule (I) et X₂ est un groupe partant, et, pour obtenir un composé de formule (I), soit
a2) la réaction d'un composé de formule (IV) avec un composé de formule 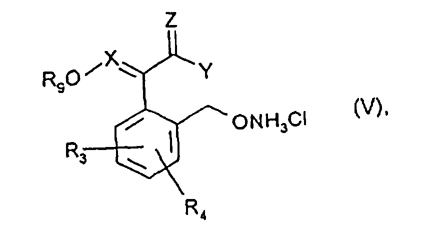 dans laquelle X, Y, Z, R₃, R₄ et R₉ sont tels que définis pour la formule (I), ou bien, selon le cas, un tautomère de ce composé, dans chaque cas sous la forme libre ou sous la forme de sel ; ou bien
b2) la conversion du composé de formule (IV) en un composé de formule 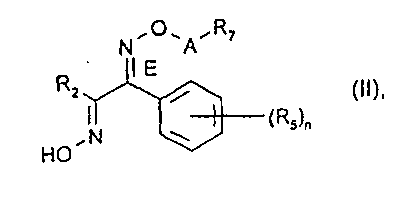 dans laquelle A, R₂, R₅, R₇ et n sont tels que définis pour la formule (I) et la double liaison C=N marquée de la lettre E a la configuration E, ou d'un tautomère possible de ce composé, dans chaque cas sous la forme libre ou sous la forme d'un sel, par réaction du composé de formule (IV) avec l'hydroxylamine ou un sel d'hydroxylamine ; et
a1) la réaction d'un composé de formule (II) avec un composé de formule 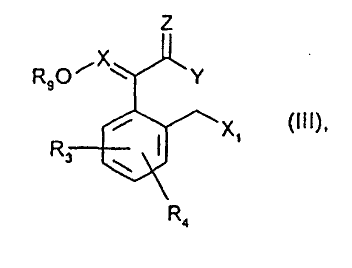 dans laquelle X, Y, Z, R₃, R₄ et R₉ sont tels que définis pour la formule (I) et X₁ est un groupe partant, ou un tautomère de ce composé, dans chaque cas sous la forme libre ou sous la forme de sel.

2. Procédé suivant la revendication 1, destiné à la préparation d'un composé de formule (I), qui comprend la réaction d'un composé de formule (II) avec un composé de formule (III).

3. Procédé suivant la revendication 2, dans lequel est utilisé un composé de formule (III) dans laquelle X₁ est un halogène.

4. Procédé suivant la revendication 1, destiné à la préparation d'un composé de formule (I), qui comprend la réaction du composé de formule (IV), avec un composé de formule (V).

5. Procédé suivant la revendication 1, destiné à la préparation d'un composé de formule (I), qui comprend la réaction du composé de formule (VI) avec un composé de formule (VII), et ou bien la réaction du composé pouvant être ainsi obtenu, de formule (IV), conformément au procédé suivant la revendication 4, ou bien la réaction de ce composé avec l'hydroxylamine ou un sel d'hydroxylamine, selon le cas en présence d'un catalyseur basique ou d'un catalyseur acide, puis la réaction du composé pouvant être ainsi obtenu, de formule (II), conformément au procédé selon la revendication 2.

6. Procédé suivant la revendication 5, dans lequel on utilise un composé de formule (VII) dans laquelle X₂ est un halogène.

7. Procédé suivant la revendication 5, dans lequel on utilise un composé de formule (VII) dans laquelle X₂ est le chlore.

8. Procédé suivant la revendication 1, pour la préparation d'un composé de formule (I), qui comprend la réaction du composé de formule (VIII) avec un nitrite d'alkyle en C₁ à C₆, puis la réaction du composé pouvant être ainsi obtenu, de formule (VI), conformément au procédé selon la revendication 5.

9. Procédé de préparation d'un composé de formule 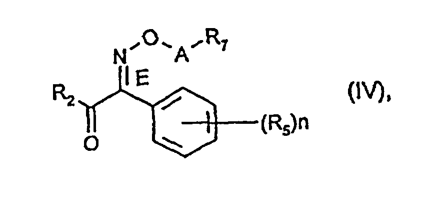 dans laquelle A, R₂, R₅, R₇ et n sont tels que définis pour la formule (I) dans la revendication 1 et la double liaison C=N marquée de la lettre E a la configuration E, qui comprend
c) la réaction d'un composé de formule (VIII) tel que défini dans la revendication 1 avec un nitrite d'alkyle en C₁ à C₆ en vue d'obtenir un composé de formule (VI) tel que défini dans la revendication 1 ; et
b1) la conversion du composé de formule (VI) en un composé de formule (IV) tel que défini ci-dessus par réaction du composé de formule (VI) avec un composé de formule (VII) tel que défini dans la revendication 1.

10. Procédé suivant la revendication 9, dans lequel on utilise un composé de formule (VII) dans laquelle X₂ est un halogène.

11. Procédé suivant la revendication 10, dans lequel on utilise un composé de formule (VII) dans laquelle X₂ est le chlore.

12. Procédé de préparation d'un composé de formule 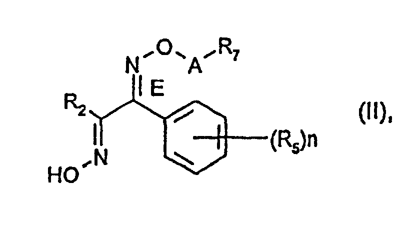 dans laquelle A, R₂, R₅, R₇ et n sont tels que définis pour la formule (I) dans la revendication 1 et la double liaison C=N marquée de la lettre E a la configuration E, qui comprend
c) la réaction d'un composé de formule (VIII) tel que défini dans la revendication 1 avec un nitrite d'alkyle en C₁ à C₆ en vue d'obtenir un composé de formule (VI) tel que défini dans la revendication 1 ;
b1) la conversion du composé de formule (VI) en un composé de formule (IV) tel que défini dans la revendication 1 par réaction du composé de formule (VI) avec un composé de formule (VII) tel que défini dans la revendication 1 ; et
b2) la conversion du composé de formule (IV) en un composé de formule (II) par réaction du composé de formule (IV) avec l'hydroxylamine ou un sel d'hydroxylamine.

13. Procédé suivant la revendication 12, dans lequel la réaction de l'étape b2) est conduite en présence de chlorhydrate d'hydroxylamine.

14. Procédé de préparation d'un composé de formule 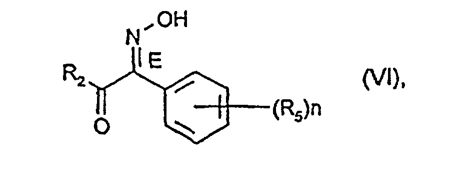 dans laquelle R₂, R₅ et n sont tels que définis pour la formule (I) dans la revendication 1 et la double liaison C=N marquée de la lette E a la configuration E, qui comprend la réaction du composé de formule (VIII) tel que défini dans la revendication 1 avec un nitrite d'alkyle en C₁ à C₆.

15. Procédé suivant la revendication 14, dans lequel la réaction est conduite en présence d'une base.

16. Procédé suivant la revendication 15, dans lequel la réaction est conduite en présence d'une base choisie dans le groupe consistant en hydroxydes, hydrures, amidures, alcanolates, acétates, carbonates, dialkylamidures et alkylsilylamidures de métaux alcalins et de métaux alcalino-terreux.

17. Procédé suivant la revendication 16, dans lequel la base est le méthanolate de sodium.

18. Procédé suivant la revendication 14, dans lequel la réaction est conduite en présence d'un solvant ou d'un diluant ou d'un mélange de solvants ou de diluants.

19. Procédé suivant la revendication 18, dans lequel le solvant est choisi dans le groupe consistant en méthanol, éthanol, propanol, isopropanol, butanol, éthylène-glycol et glycérol.

20. Procédé suivant la revendication 19, dans lequel la réaction est conduite dans du méthanol.
